# EUROPEAN PATENT SPECIFICATION

(11) **EP 3 892 216 B1**
(45) Date of publication and mention of the grant of the patent: **25.06.2025**
(21) Application number: 21167757.0
(22) Date of filing: 10.04.2021
(51) Int. Cl.: A61B 18/04, A61B 18/00

(54) **SYSTEM FOR TREATMENT OF RESPIRATORY INFECTIONS AND LUNG CANCER WITH COLD ATMOSPHERIC PLASMA**
SYSTEM ZUR BEHANDLUNG VON ATEMWEGSINFEKTIONEN UND LUNGENKREBS MIT KALTEM ATMOSPHÄRISCHEM PLASMA
SYSTÈME DE TRAITEMENT DES INFECTIONS RESPIRATOIRES ET DU CANCER DU POUMON PAR PLASMA ATMOSPHÉRIQUE FROID

(30) Priority: 10.04.2020 US 202063008510 P; 15.04.2020 US 202063010565 P; 23.04.2020 US 202063014657 P; 08.05.2020 LU 101785; 02.06.2020 US 202063033561 P
(43) Date of publication of application: 13.10.2021
(73) Proprietor: Jerome Canady Research Institute for Advanced Biological and Technological Sciences, Takoma Park MD 20912 (US)
(72) Inventor: Canady, Dr. Jerome, Takoma Park, MD, 20912 (US); Zhuang, Taisen, Takoma Park, MD, 20912 (US); Cheng, Xiaoqian, Takoma Park, MD, 20912 (US); Murthy, Saravana, Takoma Park, MD, 20912 (US); Sokolovski, Evgueni, Takoma Park, MD, 20912 (US); Yan, Feng, Takoma Park, MD, 20912 (US); Sumanasena, Buddika, Takoma Park, MD, 20912 (US); Canady, Cheffren, Takoma Park, MD, 20912 (US); McQueen, Dr. Jerome, Takoma Park, MD, 20912 (US)
(74) Representative: Tegethoff, Sebastian

(56) References cited:
- EP-A1- 2 682 063
- WO-A1-2018/189174
- US-A1- 2012 064 016
- US-A1- 2013 068 226
- US-A1- 2014 276 784
- US-A1- 2015 038 790
- US-B1- 8 460 283

## Description

The present invention relates to systems for using cold atmospheric plasma to treat respiratory infections, lung cancer, pneumonia, or other cancers of the respiratory system. The methods disclosed in the following description are not part of the claimed subject-matter.

Plasma medicine has qualified as a new scientific field after intense research effort in low-temperature or cold atmospheric plasma applications. Keidar M, Beilis II, "Plasma Engineering: application in aerospace, nanotechnology and bionanotechnology," Oxford: Elsevier; 2013. It is known that cold atmospheric plasmas ("CAP") produce various chemically reactive species including reactive oxygen species (ROS) and reactive nitrogen species (RNS). Chauvin, J., Judée, F., Yousfi, M. et al., "Analysis of reactive oxygen and nitrogen species generated in three liquid media by low temperature helium plasma jet," Sci Rep 7, 4562 (2017). CAP is a cocktail containing ROS and RNS in combination with transient electric fields, UV and charged species.

CAP has already been proven to be effective in wound healing, skin diseases, hospital hygiene, sterilization, antifungal treatments, dental care, and cosmetics targeted cell/tissue removal. One of the most recent applications of CAP is in cancer therapy. M. Keidar, et al., "Cold plasma selectivity and the possibility of a paradigm shift in cancer therapy," British Journal of Cancer (2011) 105(9), 1295 - 1301. As a near-room temperature ionized gas, cold atmospheric plasma (CAP) has demonstrated its promising capability in cancer treatment by causing the selective death of cancer cells *in vitro.* See, Yan D, Sherman J H and Keidar M, "Cold atmospheric plasma, a novel promising anticancer treatment modality," Oncotarget. 8 15977-15995 (2017); Keidar M, "Plasma for cancer treatment," Plasma Sources Sci. Technol. 24 33001 (2015); Hirst A M, Frame F M, Arya M, Maitland N J and O'Connell D, "Low temperature plasmas as emerging cancer therapeutics: the state of play and thoughts for the future," Tumor Biol. 37 7021-7031 (2016). The CAP treatment on several subcutaneous xenograft tumors and melanoma in mice has also demonstrated its potential clinical application. See, Keidar M, Walk R, Shashurin A, Srinivasan P, Sandler A, Dasgupta S, Ravi R, Guerrero-Preston R and Trink B, "Cold plasma selectivity and the possibility of a paradigm shift in cancer therapy," Br. J. Cancer. 105 1295-301 (2011); Chernets N, Kurpad D S, Alexeev V, Rodrigues D B and Freeman T A, "Reaction chemistry generated by nanosecond pulsed dielectric barrier discharge treatment is responsible for the tumor eradication in the B16 melanoma mouse model," Plasma Process. Polym. 12 1400-1409 (2015).

Additionally, various experiments have been performed in connection with the effect of CAP on viruses. In J. Zimmerman, et al., "Effects of cold atmospheric plasmas on adenoviruses in solution," J. Phys., D: 44 (2011) 505201, the authors reported successful inactivation of adenovirus, a non-enveloped double stranded DNA virus, in a solution using a surface micro-discharge technology operating in air. In X. Su et al., "Inactivation Efficacy of Nonthermal Plasma-Activated Solutions Against Newcastle Disease Virus," Applied and Environmental Microbiology, May 2018, vol. 84, issue 9, the authors reported on their investigation of the inactivation efficacy of Newcastle disease virus by non-thermal plasma-activated solutions. In T. Xie, et al., "Inactivation of airborne viruses using a packed bed non-thermal plasma reactor," J. Phys. Appl. Phys. 52 (2019), the authors reported on their study of the effectiveness of a packed bed dielectric barrier discharge (DBD) NTP reactor to inactivate bacteriophage MS2 in aerosols. See also, U.S. Published Patent Application No. 2020/0016286, entitled "Production of Immune-response Stimulating Aerosols by Non-thermal Plasma Treatment of Airborne Pathogens."

Several different systems and methods for performing Cold Atmospheric Plasma (CAP) treatment have been disclosed. For example, U.S. Patent No. 10,213,614 discloses a two-electrode system for CAP treatement of cancer cells.

Another exemplary Cold Atmospheric Plasma system is disclosed in U.S. Patent No.9,999,462. The disclosed system has two units, namely a Conversion Unit (CU) and a Cold Plasma Probe (CPP). The Conversion Unit is connected to high frequency electrosurgical generator (ESU) output and converts the ESU signal to a signal appropriate for performing cold atmospheric plasma procedures. The Cold Plasma Probe is connected to the Conversion Unit output. At the end of the Cold Plasma Probe cold plasma is produced and is thermally harmless to living tissue, i.e., it cannot cause burns to the tissue. This cold plasma, however, is deadly for cancer cells while leaving normal cells unaffected. The disclosed Cold Plasma Conversion Unit is unique in that it utilizes a high voltage transformer to up-convert the voltage (1.5-50 kV), down-convert the frequency (<300 kHz), and down-convert the power (<30 W) of the high-voltage output from an electrosurgical unit (U.S. Patent No. 9,999,462).

Additional research has shown that these CAP systems can be used to stimulate media, which then can be used for cancer treatment. For example, U.S. Patent No. 10,479,979, discloses a method for preparing a CAP stimulated media for use in cancer treatment. Another method for preparing CAP stimulated media is disclosed in U.S. Published Patent Application No. 2019/0279849.

Further, various systems and methods for controlling gas flow and an integrated gas-assisted electrosurgical generator having a graphical user interface is disclosed in WO2018/191265, entitled "Electrosurgical Gas Control Module" and WO2019199281, entitled "Gas Enhanced Electrosurgical Generator."

A variety of medical ventilator systems have been disclosed. Medical ventilators typically have a source of pressurized oxygen, which is fluidly connected to a patient through a conduit. For example, U.S. Patent No. 10,350,374 discloses a medical system having a ventilator coupled to a breaching circuit. Some ventilator systems add means for monitoring patient data. For example, U.S. Patent No. 8,554,298 discloses systems and methods for managing ventilation of a patient being ventilated by a medical ventilator, and in particular, for integrating oximeter data with the medical ventilator. Another example is U.S. Published Patent Application No. 20150034082, which discloses a ventilator-extracorporeal membrane gas-exchange (ECGE) system. Yet another example is U.S. Published Patent Application No. 20170164873, which discloses a medical ventilator with a pneumonia and pneumonia bacterial disease analysis function by using gas recognition.

Still other systems include means for supplying a medical gas with a ventilator. U.S. Published Patent Application No. 2013/0092159 discloses a method and device for supplying at least one medical gas to a patient receiving artificial respiration with the aid of a ventilator. A gas mixture provided by a respiratory gas flow of a ventilator and a medical gas added to the flow are supplied to a connecting piece, such as a Y-piece or Y-connector from which a patient feed line leads to the mechanically ventilated patient and from which a further line branches off. Via this further line at least the gas exhaled by the patient and the proportion of the respiratory gas introduced to the first line by the ventilator and the medical gas fed into the first line which have not been inhaled by the patient are discharged via the second line. For example, U.S. Published Patent Application No. 20150059743 discloses a ventilator for supplying a mixed gas of oxygen and a medical gas other than oxygen to a patient. Other relevant prior arts are disclosed in US 2012/064016 A1, US 2015/038790 A1, US 8 460 283 B1 and WO 2018/189174 A1.

### SUMMARY OF THE INVENTION

In a preferred embodiment, the present invention is a system for using cold atmospheric plasma to treat respiratory infections or cancers of the respiratory system, and, in particular, to treat patients having COVID-19.

In a preferred embodiment, the present invention is a system for performing plasma treatment of respiratory infections. "Plasma treatment of respiratory infections" as used herein refers to the use of a plasma to generate reactive species to be delivered to a patient's respiratory system. The system has a source of a carrier gas, a humidifier connected to the source of a carrier gas, a source of a feed gas, a humidifier connected to the source of a feed gas, a plasma generator configured to plasmatize the carrier gas into a plasma, a mixer and a fluid delivery member connected to an output of the mixer for delivering reactive species generated in the mixer to a patient. The mixer has an interior chamber formed from a dielectric, an active electrode inside the interior chamber and connected to an electrical output of the plasma generator, and an outer electrode connected to a ground, wherein the mixer has a first fluid input port connected to the source of a carrier gas and a second fluid input connected to the source of a feed gas. The structure of the mixer forms a dielectric barrier discharge system for generating plasma. The carrier gas may comprise at least one of helium, argon, nitrogen and oxygen. The delivery member, for example, may be endobronchial tube, a nasal cannula or a face mask. The source of a feed gas comprises one of a ventilator and a continuous positive airway pressure device and may comprise a mixture of air and oxygen.

The plasma generator preferably operates with a frequency in the range of 10kHz to 200kHz and an output peak voltage in the range of 3kV to 6kV. In a preferred embodiment, the plasma generator generates electrical energy having a frequency within 5kHz of one of 40kHz, 100kHz and 200 kHz. In another preferred embodiment, the plasma generator generates electrical energy having a frequency of 122kHz. The plasma generator may be a combination a high frequency electrosurgical generator and a low frequency converter. The plasma generator may have a power module, a CPU for controlling the power module, a memory connected to the CPU and a power supply connected to the CPU. Still further, the plasma generator may have a touchscreen display, a controller connected to the touchscreen display and a graphical user interface configured to display data on the touchscreen display and receive input from a user through the touch-screen display. The plasma generator further may have a gas module. The source of a carrier gas may be connected to the gas module and the gas module controls a flow of the carrier gas to the mixer. The first humidifier may be connected between the gas module and the mixer or may be connected between the gas module and the source of a carrier gas.

In a preferred embodiment, the first humidifier is configured to humidify a carrier gas flowing from the source of a carrier gas to at least 70% humidity and the second humidifier is configured to humidify a feed gas flowing from the source of a feed gas to at least 50% humidity. For example, the first humidifier is configured to humidify a carrier gas flowing from the source of a carrier gas to 100% humidity and the second humidifier is configured to humidify a feed gas flowing from the source of a feed gas to at least 50% humidity.

In another embodiment, the present invention is a system for performing plasma treatment of respiratory system. The system has an electrical energy generator configured to generate electrical energy to plasmatize a carrier gas into a plasma and a dielectric barrier discharge ("DBD") mixer. The DBD mixer has an interior chamber formed from a dielectric, the interior chamber having a first input configured to fluidly connect to a source of a humidified carrier gas, a second input configured to connect to a source of a humidified feed gas, and an output configured to connect to a delivery member, an active electrode inside the interior chamber and connected to an electrical output of the electrical energy generator, and an outer electrode connected to a ground. A plasma is generated in the interior chamber when electrical energy is supplied from the electrical energy generator to the interior electrode while both humidified feed gas and humidified carrier gas flow into the interior chamber. The system further may have a first humidifier fluidly connected to the first input of the chamber in the dielectric barrier discharge assembly and a second humidifier fluidly connected to the second input of the chamber in the dielectric barrier discharge assembly. Still further, the system may have a source of un-humidified helium fluidly connected to an input of the first humidifier and a source of un-humidified air fluidly connected to an input of the second humidifier.

Still other aspects, features, and advantages of the present invention are readily apparent from the following detailed description, simply by illustrating preferable embodiments and implementations. The present invention is also capable of other and different embodiments and its several details can be modified in various obvious respects, all without departing from the scope of the present invention. Accordingly, the drawings and descriptions are to be regarded as illustrative in nature and not as restrictive. Additional objects and advantages of the invention will be set forth in part in the description which follows and in part will be obvious from the description or may be learned by practice of the invention. The invention is defined by the appended claims.

### BRIEF DESCRIPTION OF THE DRAWINGS

For a more complete understanding of the present invention and the advantages thereof, reference is now made to the following description and the accompanying drawings, in which:
FIG. 1A is a block diagram of a cold atmospheric plasma system having a CAP Joint Mixer for treatment of respiratory infections in accordance with a preferred embodiment in which both a carrier gas and a feed gas are humidified.
FIG. 1B is an illustration of a system layout for the dielectric barrier discharge assembly and connecting hoses of FIG. 1A.
FIG. 1C is a close-up view of a system layout for the dielectric barrier discharge assembly and connecting hoses of FIG. 1A.
FIG. 1D is an exploded view of a system layout for the dielectric barrier discharge assembly and connecting hoses of FIG. 1A.
FIG. 1E is a close-up exploded view of a system layout for the dielectric barrier discharge assembly and connecting hoses of FIG. 1A.
FIG. 1F is a close-up cross-sectional view of a system layout for the dielectric barrier discharge assembly and connecting hoses of FIG. 1A.
FIG. 2A is an isometric view of a preferred embodiment of a CAP joint mixer or dielectric barrier discharge (DBD) assembly in accordance with the preferred embodiments of the present invention.
FIG. 2B is An exploded view of a preferred embodiment of a CAP joint mixer or dielectric barrier discharge (DBD) assembly in accordance with the preferred embodiments of the present invention.
FIG. 2C is a partial cross-sectional view of a preferred embodiment of a CAP joint mixer or dielectric barrier discharge (DBD) assembly in accordance with the preferred embodiments of the present invention.
FIG. 2D is a front view of a preferred embodiment of a CAP joint mixer or dielectric barrier discharge (DBD) assembly in accordance with the preferred embodiments of the present invention.
FIG. 2E is a back view of a preferred embodiment of a CAP joint mixer or dielectric barrier discharge (DBD) assembly in accordance with the preferred embodiments of the present invention.
FIG. 2F is a top view of a preferred embodiment of a CAP joint mixer or dielectric barrier discharge (DBD) assembly in accordance with the preferred embodiments of the present invention.
FIG. 2G is a bottom view of a preferred embodiment of a CAP joint mixer or dielectric barrier discharge (DBD) assembly in accordance with the preferred embodiments of the present invention.
FIG. 2H is a left side view of a preferred embodiment of a CAP joint mixer or dielectric barrier discharge (DBD) assembly in accordance with the preferred embodiments of the present invention.
FIG. 2I is a right side view of a preferred embodiment of a CAP joint mixer or dielectric barrier discharge (DBD) assembly in accordance with the preferred embodiments of the present invention.
FIG. 3A is a block diagram of a cold atmospheric plasma generator of a preferred embodiment of the present invention.
FIG. 3B is a block diagram of a plasma generator of an alternate preferred embodiment of the present invention.
FIG. 3C is a block diagram of a plasma generator of another alternate preferred embodiment of the present invention.
FIG. 3D is a block diagram of an integrated gas-enhanced electrosurgical generator having a plurality of gas modules of another alternate preferred embodiment of the present invention.
FIG. 4 is a perspective view of an integrated gas-enhanced electrosurgical generator of a preferred embodiment of the present invention.
FIG. 5 is a flow diagram illustrating a method for treatment a respiratory infection in which both a carrier gas and a feed gas are humidified.
FIG. 6 is a flow diagram illustrating a method for treatment a respiratory infection in which both a carrier gas and a feed gas are humidified and in which the CAP generator sweeps through a plurality of settings during a single treatment.
FIG. 7A is a block diagram of a cold atmospheric plasma system having a CAP Joint Mixer for treatment of respiratory infections in accordance with a second preferred embodiment in which a carrier gas is humidified.
FIG. 7B is a flow diagram illustrating a method for treatment of a respiratory infection in accordance with a second preferred embodiment in which a carrier gas is humidified.
FIG. 8A is a block diagram of a cold atmospheric plasma system having a CAP Joint Mixer for treatment of respiratory infections in accordance with a third preferred embodiment in which a feed gas is humidified.
FIG. 8B is a flow diagram illustrating a method for treatment of a respiratory infection in accordance with a third preferred embodiment in which a feed gas is humidified.
FIG. 9 is a block diagram of a cold atmospheric plasma system having a CAP Joint Mixer for endoscopic or laparoscopic uses in accordance with a preferred embodiment.
FIGs. 10A and 10B are graphs of the concentrations of H₂O₂ and NO₂⁻ with the cold atmospheric plasma system for treatment of respiratory infections of FIG. 1C.
FIG. 10C is a graph of viability of A549 cells treated by the present invention with humidified O₂ and air mixture and various O₂ percentage for up to 4 minutes.
FIG. 10D is a graph of viability of A549 cells treated by the present invention with humidified Air/O₂ mixture and dry He with 24% O₂ for up to 17 minutes.
FIG. 10E is a graph of viability of A549 cells treated by the present invention with humidified helium and dry O₂ and air mixture (with 24% O₂) for 17 minutes.
FIG. 10F is a graph of viability of A549 cells treated by a system in accordance with the present invention for 5 or 10 minutes with humidified Air/O₂ mixture (with 24% O₂) and various helium humidity.
FIG. 10G is a graph of viability of lung cancer cells A549 treated by a system in accordance with a preferred embodiment of the present invention at 48-hour post treatment with humidified Air/O₂ mixture and He + 0-100% humidity for 1-5 min.
FIG. 10H is a graph of viability of lung cancer cells A549 treated by by a system in accordance with a preferred embodiment of the present invention at 48-hour post treatment with dry Air/O₂ mixture and He + 0-100% humidity for 1-5 min.
FIG. 11 is a block diagram of a cold atmospheric plasma system having a CAP Joint Mixer for treatment of respiratory infections in accordance with another preferred embodiment in which a carrier gas, a feed gas and a third gas are humidified.

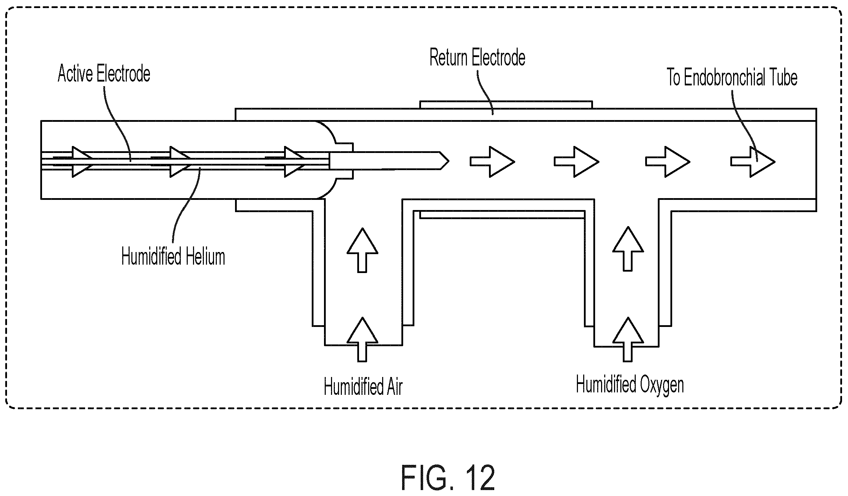

FIG. 12 is a diagram of an alternate embodiment of a CAP Joint Mixer in which supplies of air and oxygen enter the CAP Joint Mixer in different locations such that at least one of the air and oxygen enter the CAP Joint Mixer downstream of the inner electrode.
FIG. 13A is a graph of viability of A549 treated by a by a system in accordance with a preferred embodiment of the present invention with humidified Air/O₂ mixture.
FIG. 13B is a graph of viability of A549 treated by by a system in accordance with a preferred embodiment of the present invention with humidified Air and humidified O₂ separately.
FIG. 14A is a graph of ozone production rate by a system in accordance with a preferred embodiment of the present invention with humidified or dry Air/O₂ mixture and He + 0-100% humidity.
FIG. 14B is a graph of ozone production rate by a system in accordance with a preferred embodiment of the present invention with humidified Air and O₂ mixture or separately and humidified He at different voltage.
FIG. 14C is a graph of a first set of experimental results of ozone production rate by a system in accordance with a preferred embodiment of the present invention with humidified Air and O₂ mixture or separately and humidified He at different voltages ranging from 35-40V.
FIG. 14D is a graph a second set of experimental results of ozone production rate by a system in accordance with a preferred embodiment of the present invention with humidified Air and O₂ mixture or separately and humidified He at different voltages ranging from 35-40V taken on a different day that the first set.
FIG. 15A is a graph of hydrogen peroxide (H₂O₂) production rate by a system in accordance with a preferred embodiment of the present invention. PBS was treated with humidified Air/O₂ and humidified He with CAP and gas mixture (He, Air and O₂) for 8 or 15 min continuously and in intervals.
FIG. 15B is a graph of nitrite (NO₂⁻) production rate by a system in accordance with a preferred embodiment of the present invention. PBS was treated with humidified Air/O₂ and humidified He with CAP and gas mixture (He, Air and O₂) for 8 or 15 min continuously and in intervals.
FIG. 15C is a graph of Nitrate (NO₃⁻) production rate by the by a system in accordance with a preferred embodiment of the present invention. PBS was treated with humidified Air/O₂ and humidified He with CAP and gas mixture (He, Air and O₂) for 8 or 15 min continuously and in intervals.

### DETAILED DESCRIPTION OF THE PREFERRED EMBODIMENTS

Cold atmospheric-pressure plasma (CAP) generates numerous reactive oxygen species (ROS) and reactive nitrogen species (RNS), such as hydroxyl radical (^{·}OH), singlet oxygen (¹O₂), nitrogen ion (N₂⁺), atomic oxygen (O), and, as well as electrons, ions, and photons. Maximum concentration of these species can be reached with optimal amount of humidity in the gas. CAP-generated ROS and RNS can form hydrogen peroxide (H₂O₂), nitrite (NO₂⁻), nitrate (NO₃⁻), peroxynitrite (ONOO-) when interacting with biological fluid. Reactive species and radicals in the plasma phase (^{·} OH, N₂⁺,¹O₂, O) are short-lived species, whereas H₂O₂, NO₂⁻, NO₃⁻, and ONOO- in aqueous phase are long-lived species. The long-lived species will further interact with intracellular species and metabolic pathways, inducing cell apoptosis. The present invention provides a system with which a cold atmospheric plasma (non-thermal plasma) can be generated and the reactive species can be delivered to patients across much greater distances than in prior systems. While prior systems typically are used at a distance of 2-10 cm from the target tissue, the present invention delivers plasma to the patient from a distance of greater than 10 cm.

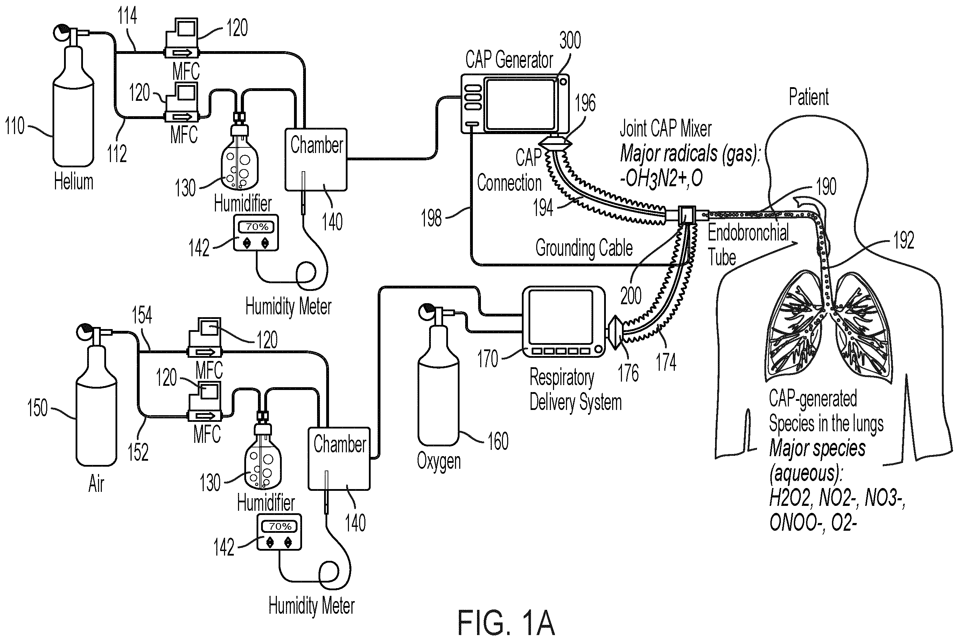

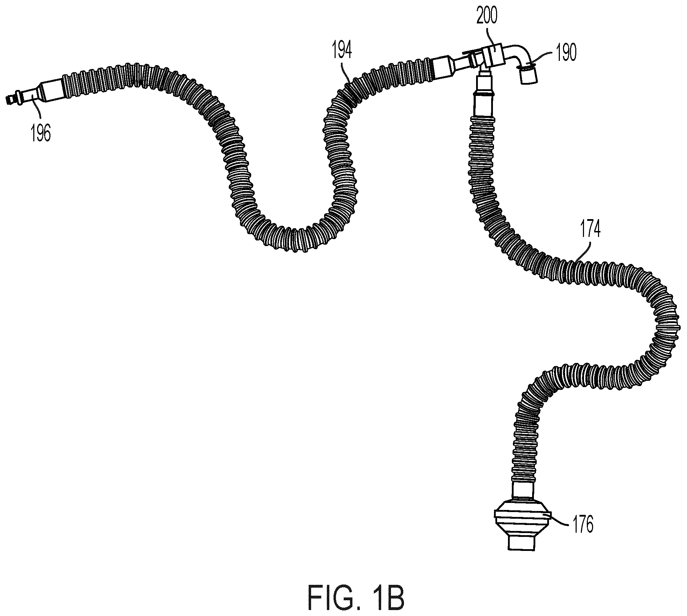

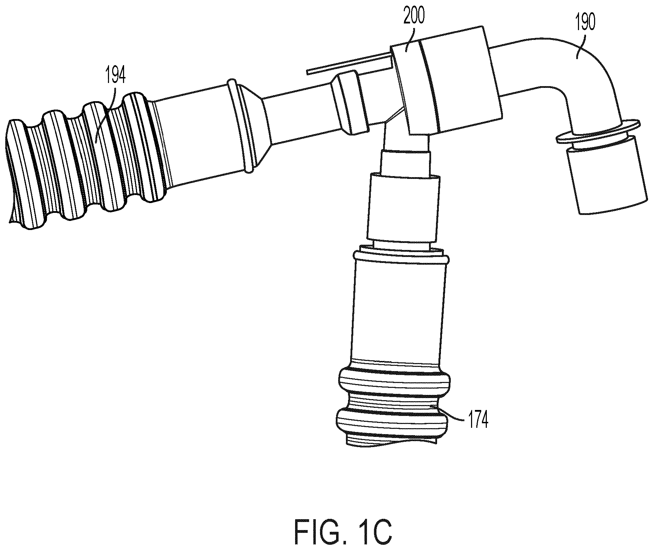

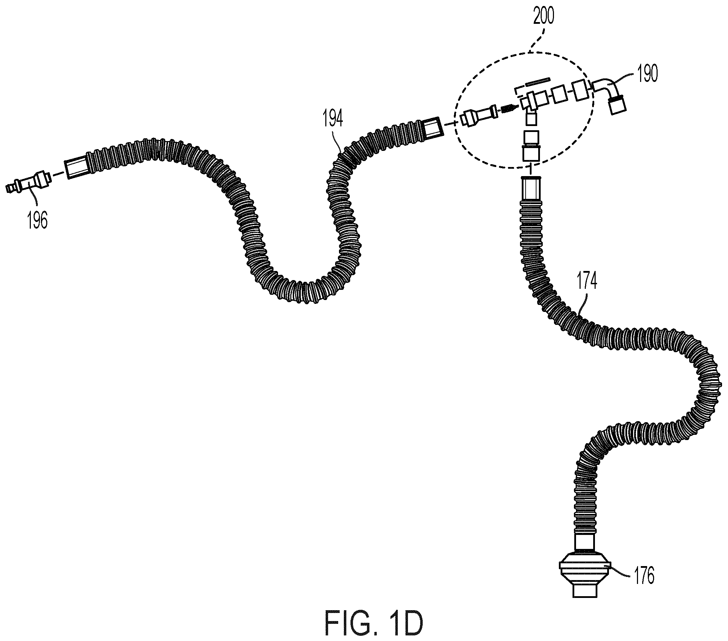

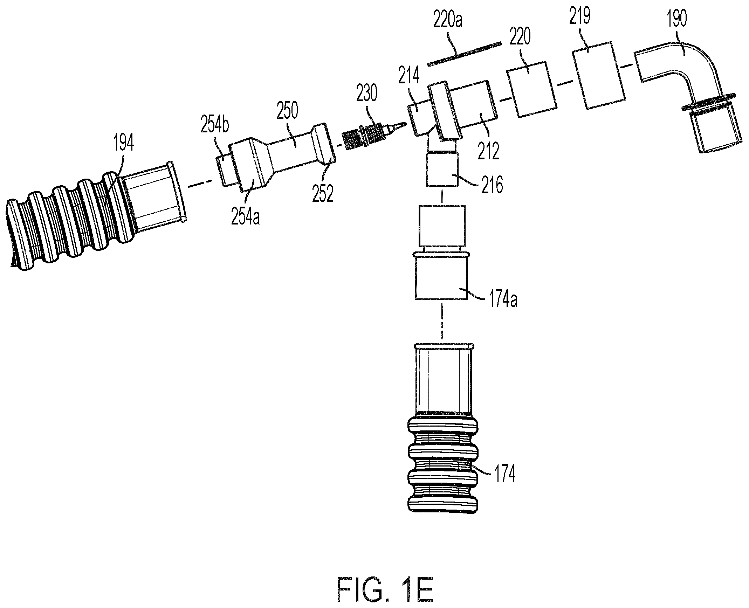

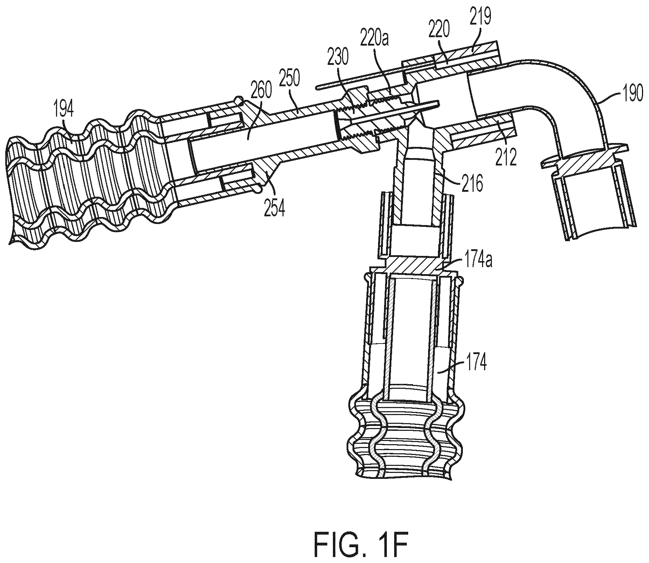

A cold atmospheric plasma system for treatment of respiratory infections in accordance with a first preferred embodiment of the present invention is described with reference to FIG. 1. In this embodiment, the carrier as is helium and the feed gas is air. A helium gas source 110 is split into two lines 112, 114, with each of the two lines controlled by a mass flow controller (MFC) 120. Line 112 is fluidly connected to a first Humidifier 130. The Helium gas flow (50 to 1000 mL/min) in line 112 is passed through an H₂O filled container (Humidifier 130) and then fed into a mixing chamber 140. The helium gas flow in the line 114 is fed directly into the mixing chamber 140. In this manner, with the mass flow controllers 120 on the lines 112, 114 a relative H₂O saturation in the gas exiting the chamber 140 can be adjusted. Adjustment of the gas flow in the two lines 112, 114 makes the overall flow rate and humidity fine tuning of the gas flow exiting the chamber 140 possible. The humidity may be in the range of 20%-100% with a preferred humidity of at least 70%. The total helium flow in this embodiment could be varied from 0.5 L/min to 5 L/min in all cases. The humidity of Helium gas in the chamber 140 is measured via calibrated High-Accuracy Humidity and Temperature Meter 142. The humidified helium gas from the chamber 140 is fed into an electrosurgical generator 300, referred to herein as a "Cold Atmospheric Plasma (CAP) Generator." A variety of electrosurgical generators are known in the art and could be used with the present invention. The gas being fed into the Cold Atmospheric Plasma (CAP) Generator 300 is referred to herein as the "carrier gas."

At the same time, an un-humidified air supply 150 (feed gas) is split into two lines 152, 154. Each line 152, 154 is controlled by a mass flow controllers (MFC) 120. Line 152 is fluidly connected to a second Humidifier 130. The air gas flow in line 152 is passed through an H₂O filled container (Humidifier 130) and then is fed into a mixing chamber 140. The air gas flow in the line 114 is fed directly into the mixing chamber 140. In this manner, with the mass flow controllers 120 on the lines 152, 154 a relative H₂O saturation in the air feed exiting the chamber 140 can be adjusted. Adjustment of the air flow in the two lines 152, 154 makes the overall flow rate and humidity fine tuning possible. The humidity may be in the range of 20%-100% with a preferred humidity of at least 70%. The humidity of air in the chamber 140 is measured via calibrated High-Accuracy Humidity and Temperature Meter 142. The humidified air from the chamber 140 and oxygen from an oxygen supply 160 are provided to a respiratory delivery system 170, such as a ventilator, CPAP machine, BIPAP machine, or other known respiratory deliver system. The respiratory delivery system 170 will mix, adjust and measure the pressure, flowrate, ratio and frequency of the patient inbreath of exhaust air, oxygen and CO2. The output of the respiratory delivery system 170 is connected to the CAP joint mixer, for example, via tubing 174 and a connector 176. In testing, humidifying the helium flow to 100% humidity and the air flow to 50% humidity proved to be effective.

The output of the CAP generator 300 and the respiratory delivery system 170 are connected to a dielectric barrier discharge (DBD) assembly 200, referred to herein as a "CAP joint mixer." A ground cable 198 connects an outer electrode of the CAP joint mixer 200 to a ground in the CAP generator 300. While the grounding cable 198 is shown separate from the tubing 194 in FIG. 1, other arrangements are possible in which the ground cable 198 is combined, for example, in a harness with the tubing 194. Due to the presence of the H₂O, the ionization of Helium and H₂O to He⁺ + e⁻ chemical reaction will happen simultaneously. The cold plasma-generated reactive species (H2O2, NO2-, NO3-, ONOO-, and 02-) are produced.

The output of the CAP joint mixer 200 is connected to a delivery member 190, which, for example, may be an endobronchial tube, oxygen CPAP (continuous positive airway pressure), BIPAP (Bilevel Positive Airway Pressure), ventilator face mask, or nasal O₂ cannula 190 to deliver reactive species 192, e.g., H2O2, NO2-, NO3-, ONOO-, and 02-, generated by the system into the patient's respiratory system.

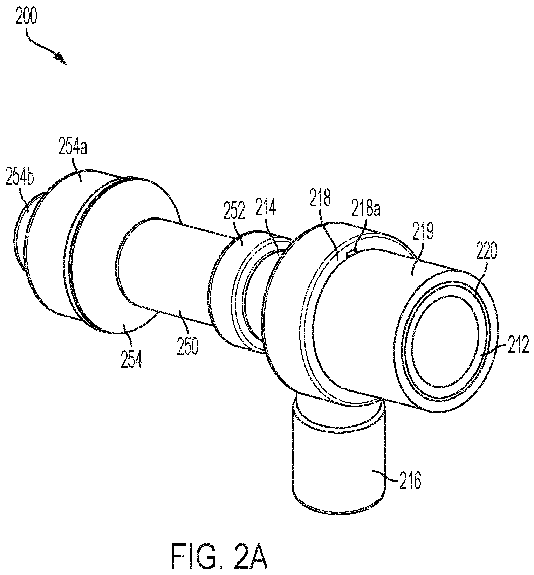

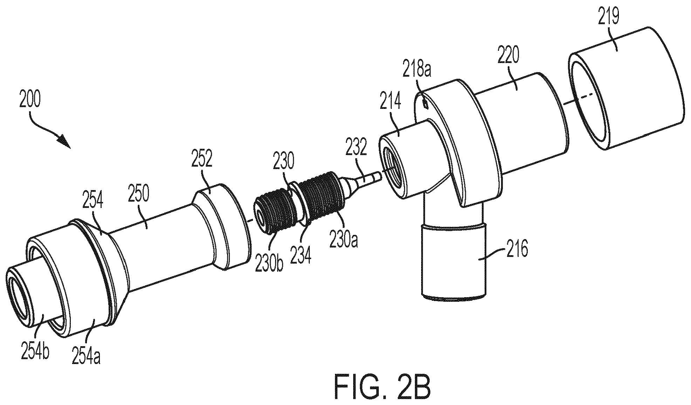

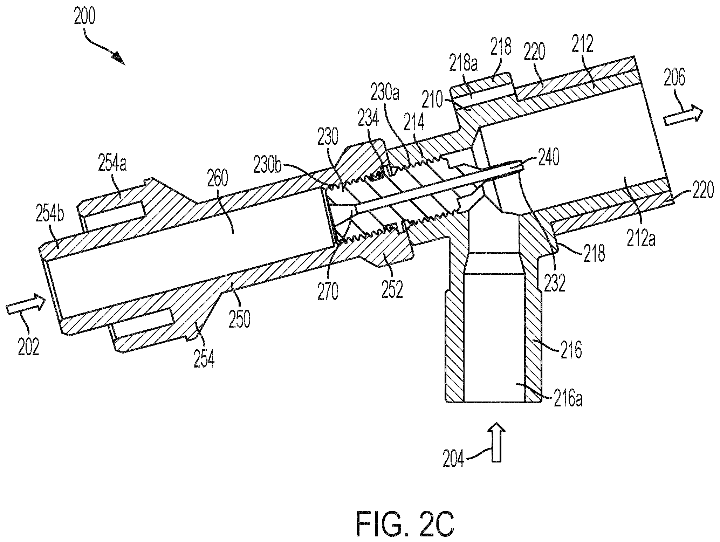

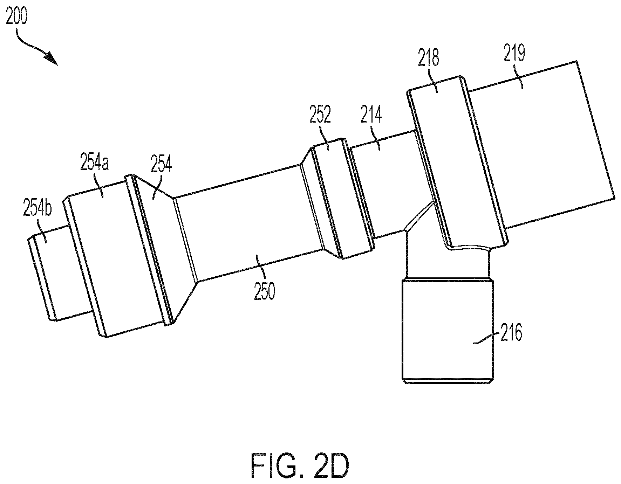

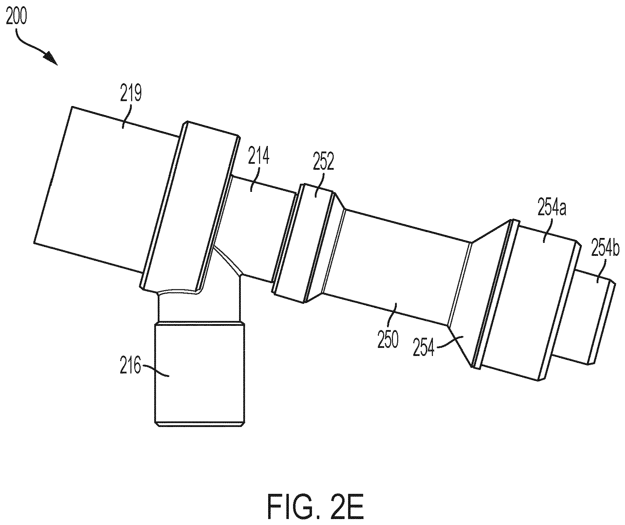

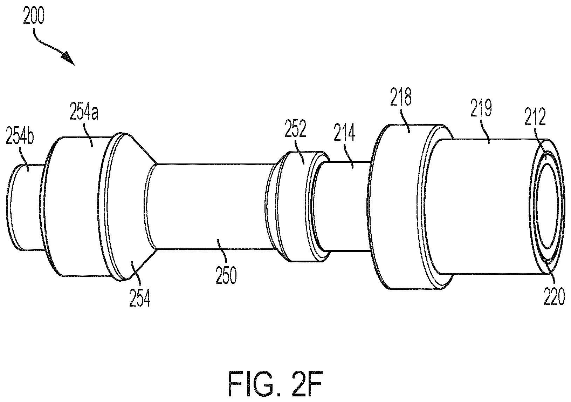

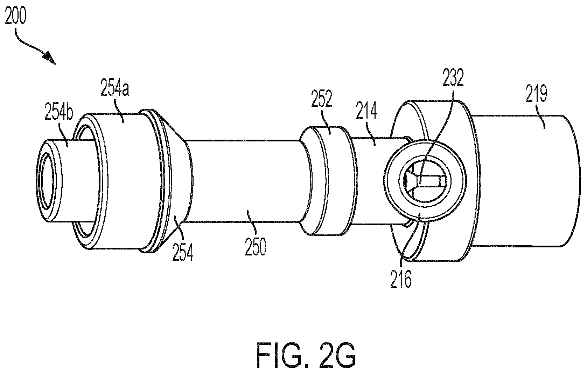

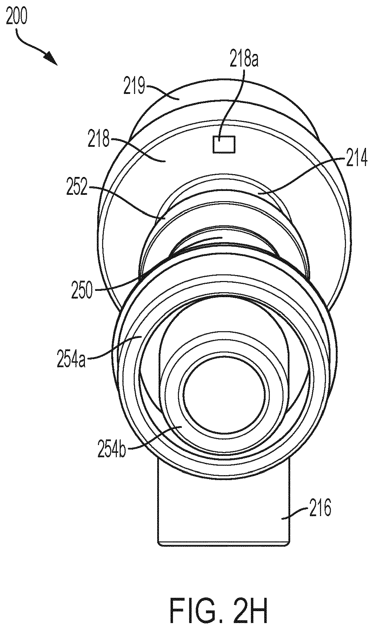

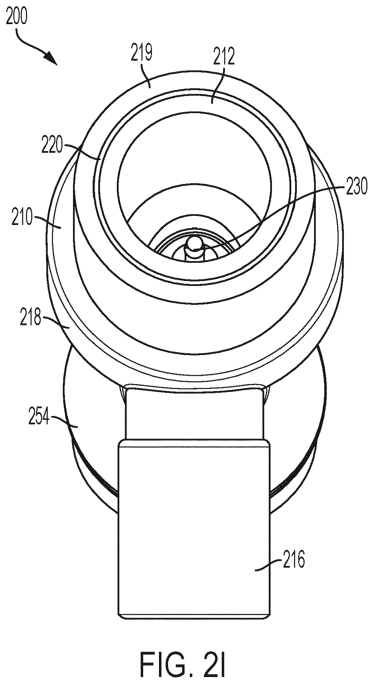

A preferred embodiment of a dielectric barrier discharge (DBD assembly or CAP Joint Mixer 200 is described with reference to FIGs. 2A-2I. The DBD assembly 200 has a first entry port 202 for receiving a flow of a first gas (e.g., a carrier gas), a second entry port 204 for receiving a second gas (e.g., a feed gas), and an exit port 206 through which gases and reactive species generated in the DBD assembly exit the assembly. The assembly has a primary housing 210 having a portion 212 forming a chamber 212a within the primary housing 210. At least the portion 212 forming the chamber 212a is a dielectric material. In a preferred embodiment, the entire primary housing 210 is formed of a dielectric material, but other embodiments are possible wherein only a portion of the primary housing 210 including the portion 212 that forms the chamber 212a is formed of a dielectric material. In still other embodiments, a dielectric material separate from the primary housing 210 may surround the chamber 212a. The portion 212 of the primary housing 210 forms the exit port 206 to which a delivery member, such as an endobracheal tube or other type of tube, may be connected. The invention is not limited to any particular type of delivery member or connection between the delivery member and the exit port 206. The primary housing 210 has a first neck or connector portion 214 forming a first input port 202 for receiving a first gas, which in this embodiment is the carrier gas (e.g., helium), and a channel leading to the chamber 212a. The interior of the neck portion 214 is threaded for receiving an interior electrode 230. The primary housing 210 further has a second neck or connector 216 forming a second input port 204 for receiving a second gas, which in this embodiment is a feed gas (e.g., an air/oxygen mixture) and a channel 216a leading to the chamber 212a. A second outer electrode 220 made of a conductive material, e.g., copper, surrounds the exterior of the dielectric forming the chamber 212a. As shown in FIGs. 2A and 2B, an outer insulating layer 219 covers the outer electrode 220. The outer insulating layer 219 is not shown in FIG. 2C. As shown in FIG. 1, the outer electrode 220 is connected to a ground. The housing 210 has a lip or ridge 218 abutting the outer electrode 220. Within an upper portion of the lip or ridge 218 is a hole channel 218a, which allows for the outer electrode 220 to be connected to a ground wire 198 (see FIG. 1) via connecting wire 220a.

The inner electrode 230 is made of a conductive material and has within it a channel 240 through which the first gas (a carrier gas) flows. The electrode 230 has a neck 232 that extends into the chamber 212a. The channel 240 extends through the neck 232 such that the first gas (the carrier gas) can flow into the chamber 212a. The exterior of the electrode 230 two threaded portions 230a, 230b and a lip or ridge 234. The threaded portion 230a engages with the threaded interior of the neck 214 of the primary housing 210 to secure the inner electrode 230 into the primary housing 210. The ridge or lip 234 of the electrode 230 provides a stop when the electrode 230 is fully threaded into the neck 214.

The dielectric barrier discharge (DBD) assembly 200 further has a secondary housing 250 having a channel within it through which the first gas (the carrier gas) can flow to the channel 240 in the inner electrode 230. The secondary housing 250 has a portion 252 having interior threaded for engaging the threaded portion 230b of the electrode 230 and thereby securing the secondary housing to the electrode 230 and the primary housing 210. The secondary housing 250 has at the end of the threaded portion 252 a recess for receiving the electrode ridge or lip 234 and abutting the neck 214 of the primary housing 210. The secondary housing 250 further has a connector structure 254, 254a, 254b for connecting to a hose or other tubing 194 and connector 196 to connect the dielectric barrier discharge (DBD) assembly to the CAP generator 300. Within the secondary housing 250 is a tube 260 through which the first gas (the carrier gas) flows. Within the tube 260 is an elongated electrode or wire that is connected to a conductive connector 270 (e.g., via solder). The conductive connector 270 abuts the inner electrode 230 and thus is electrically connected to the inner electrode 230.

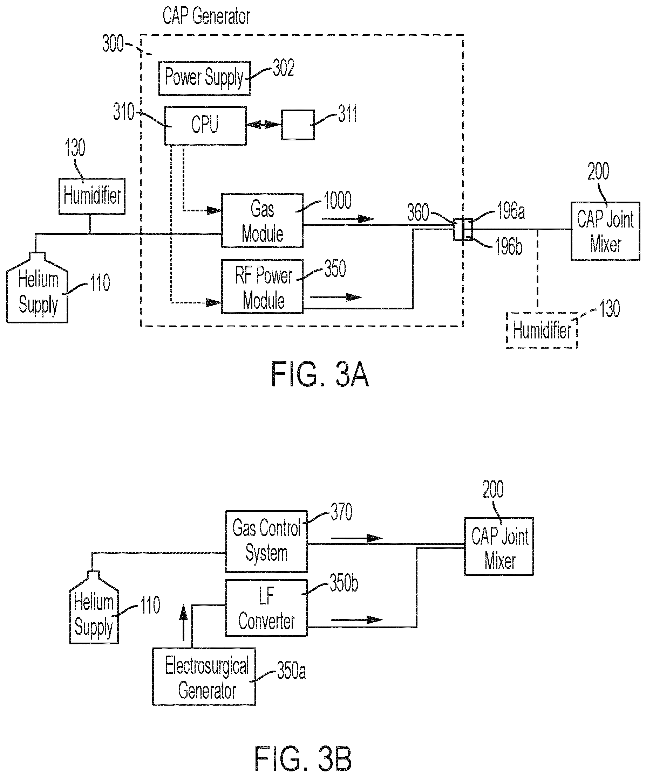

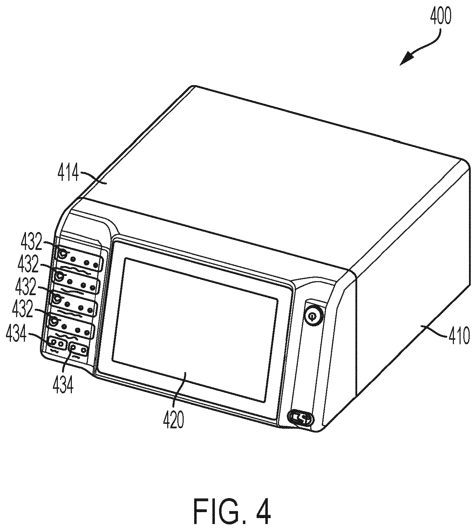

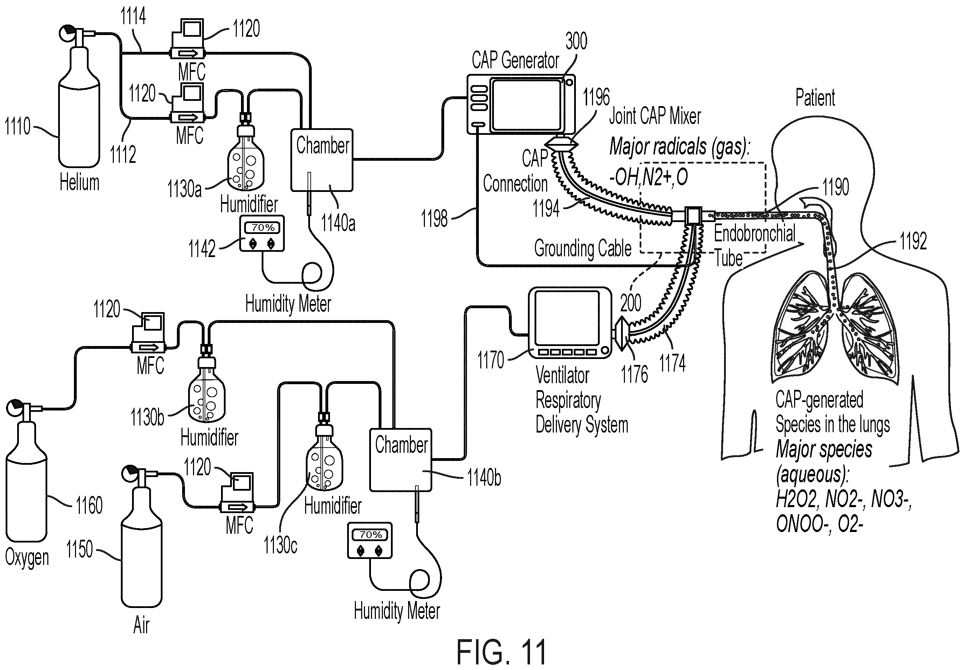

As shown in FIG. 3A, an exemplary cold atmospheric plasma (CAP) generator 300 has a power supply 302, a CPU (or processor or FPGA) 310 and a memory or storage 311. The system further has a display 420 (FIG. 4), which may be the display of a tablet computer. The CPU 310 controls the system and receives input from a user through a graphical user interface displayed on display 420. The CAP generator further has a gas control module 1000 connected to a source 310 of a CAP carrier gas such as helium to control the flow of the carrier gas to the CAP joint mixer. The CAP generator 300 further has a radio frequency (RF) power module 350 for generating radio frequency (RF) energy. The RF power module contains conventional electronics such as are known for providing RF power in electrosurgical generators. The RF Power module operates with a frequency between 10-200kHz and output peak voltage from 3kV to 6kV and preferably at a frequency near (within 25%) of 40kHz, 100kHz or 200kHz. The gas module 1000 and RF power module 350 are connected to connector 360 that allows for CAP joint mixer 200 (or a CAP applicator 1100 in FIGs. 11A and 11B) to be connected to the generator 300 via a connector having an electrical connector 196a and gas connector 196b.

As shown in FIG. 3B, other arrangements for delivery of the carrier gas and the electrical energy may be used with the invention. In FIG. 3B, a source 110 of a carrier gas (helium in this example) is provided to a gas control system 370 of any type, which supply the gas at a controlled flow rate to CAP joint mixer 200. A conventional electrosurgical generator 350a supplies high frequency (HF) energy to a low frequency converter 350b, which outputs electrical energy having a frequency in the range of 10kHz to 200kHz and an output voltage in the range of 3kV to 6Kv.

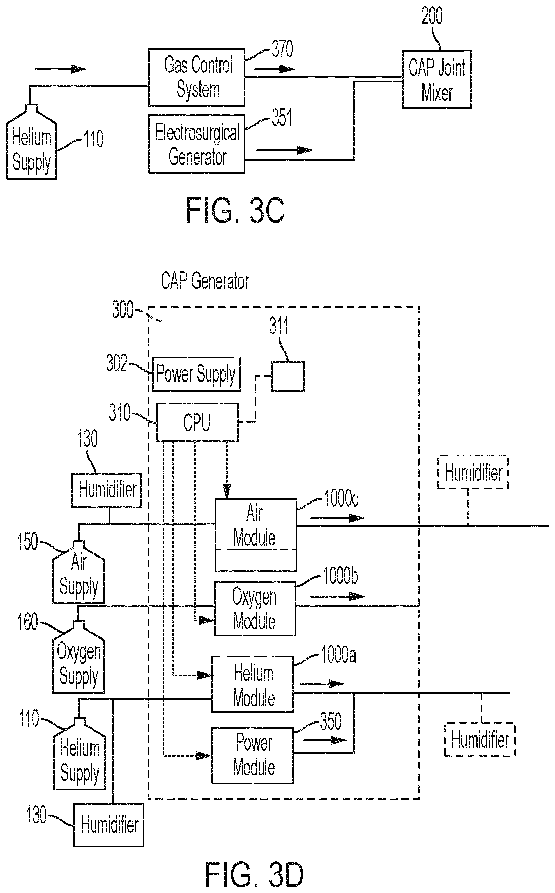

Another embodiment, shown in FIG. 3C, has a carrier gas source 110 connected to a conventional gas control system 370, which in turn is connected to the CAP joint mixer 200, and a conventional electrosurgical generator 351 also connected to the CAP joint mixer 200.

A generator housing 400 for a CAP-enabled gas-enhanced electrosurgical generator 300 in accordance with a preferred embodiment of the present invention is shown in FIG. 4. The generator housing 400 has a housing 410 made of a sturdy material such as plastic or metal similar to materials used for housings of conventional electrosurgical generators. The housing 410 has a removable cover 414. The housing 410 and cover 414 have means, such as screws, tongue and groove, or other structure for removably securing the cover to the housing. The cover 414 may comprise just the top of the housing or multiple sides, such as the top, right side, and left side, of the housing 410. The housing 410 may have a plurality of feet or legs attached to the bottom of the housing. The bottom of the housing 410 may have a plurality of vents for venting from the interior of the gas-enhanced generator.

On the face of the housing 414 there is a touch-screen display 420 and a plurality of connectors 432, 434 for connecting various accessories to the generator, such as an argon plasma probe, a hybrid plasma probe, a cold atmospheric plasma probe, or any other electrosurgical attachment. The face of the housing 410 is at an angle other than 90 degrees with respect to the top and bottom of the housing 410 to provide for easier viewing and use of the touch screen display 420 by a user. One or more of the gas control modules may be mounted within a gas-enhanced electrosurgical generator 300.

The CAP-enabled gas-assisted electrosurgical generator has a graphical user interface (GUI) for controlling the components of the system using the touch screen display 420. The graphical user interface for example, may control robotics, argon-monopolar cut/coag, hybrid plasma cut, cold atmospheric plasma, bipolar, plasma sealer, hemo dynamics or voice activation. The graphical user interface further may be used with fluorescence-guided surgery. The graphical user interface (GUI) further may be used with guided imaging such as CT, MRI, or ultrasound. The graphical user interface may communicate with RFID (such as may be found in various electrosurgical attachments) and may collect and store usage data in a storage medium. The graphical user interface communicates with the field-programmable gate array ("FPGA"), which may control an irrigation pump, insufflator, full bridge for adjusting the power output, fly back for regulating the power (DC to AC) and a foot pedal. The GUI further communicates with a database of data with associated predicted CAP settings or dosages via the CPU 310. The database storage may be internal memory or other internal storage 311 or external storage.

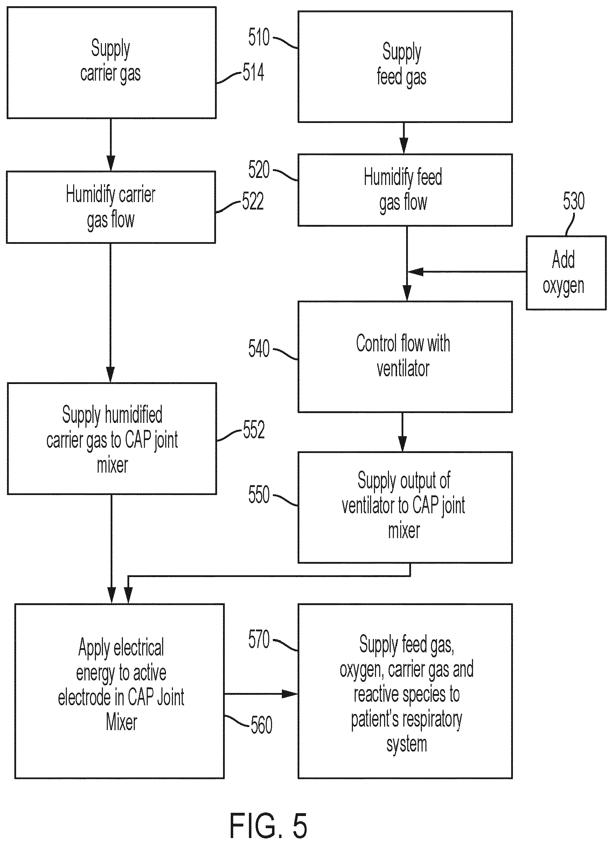

A method for treatment of a respiratory infection in which both the feed gas (air) and the carrier gas (helium) are humidified is described with reference to FIG. 5. Pressurized feed gas (air) is supplied to a humidifier 510. The pressurized air is humidified in a humidifier 520. Oxygen is added to the humidified air flow 530. The humidified air and oxygen flow is controlled by a ventilator or other respiratory delivery system 540. At the same time, a CAP carrier gas such as helium is supplied to a humidifier 514. The carrier gas is humidified in the humidifier 522. The humidified carrier gas is supplied to a CAP generator. The humidified CAP carrier gas from the CAP generator and the output of the ventilator both are supplied to a CAP joint mixer 550, 552. Electrical energy is applied to an inner electrode in the CAP joint mixer 560. The output of the CAP joint mixer is then supplied to the patient's respiratory system 570, for example, via a respiratory face mask, nasal cannula, or endobronchial tube.

In studies on the treatment of cancer using cold atmospheric plasma, it has been found that the CAP treatment decreases viability of cancer cells in a dose-dependent manner. Rowe, W., et al., "The Canady Helios Cold Plasma Scalpel Significantly Decreases Viability in Malignant Solid Tumor Cells in a Dose-Dependent Manner," Plasma, 2018. 1(1): p. 177-188.

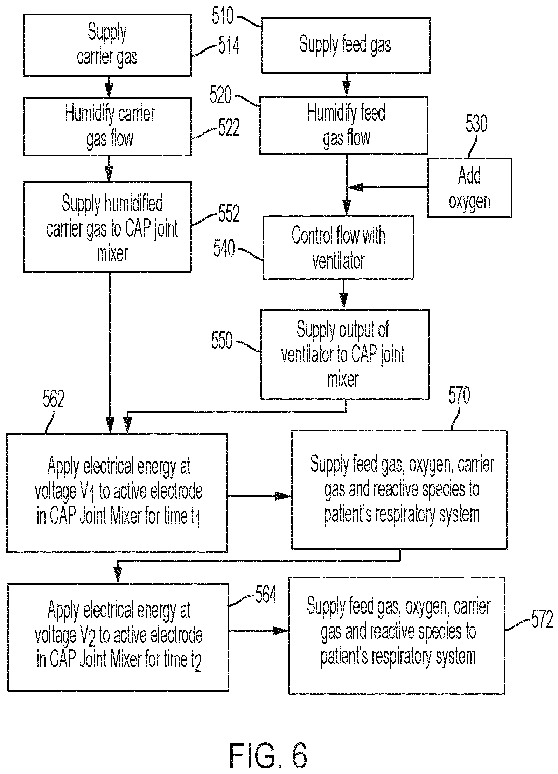

A method for treatment a respiratory infection in which both the feed gas (air) and the carrier gas (helium) are humidified is described with reference to FIG. 6. In this embodiment, rather than electrical energy being applied to the inner electrode in the CAP joint mixer at a single setting (e.g., 70 V) for the entire treatment time (step 560 in FIG. 5), the generator automatically sweeps thought a plurality of settings applying a first setting (e.g., 70 V) for a first time t₁ 562 while supplying the output to the patient 570 and then applying a second setting (e.g., 40 V) for a second time t₂ 564 while supplying the output to the patient 572.

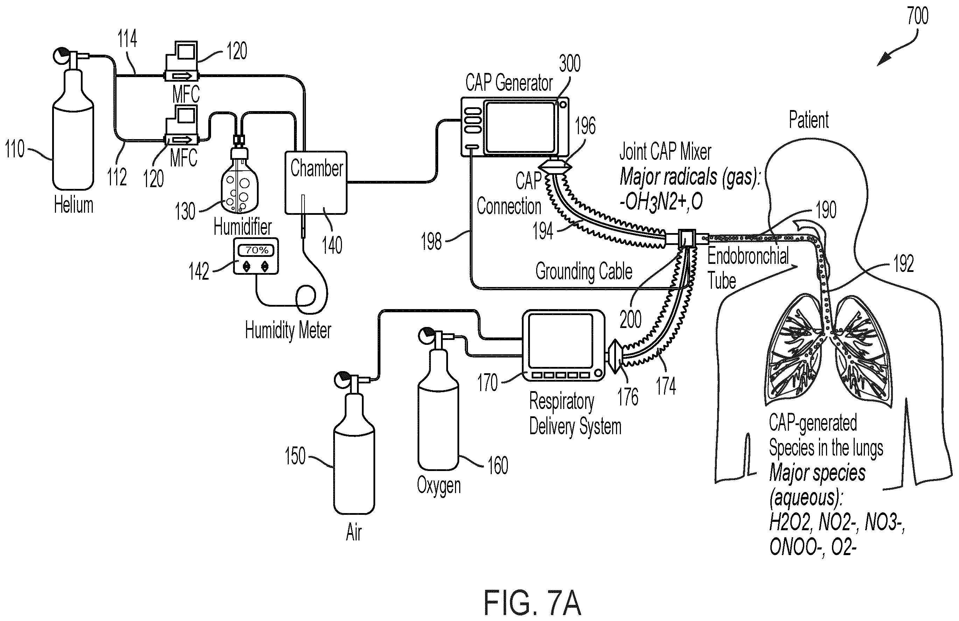

An alternative embodiment of a system 700 is referred to herein as the "Helium Gas Humidity Adjustment Setup" is described with reference to FIG. 7A. A helium gas source 110 is split into two lines 112, 114, with each of the two lines controlled by a mass flow controller (MFC) 120. The Helium gas flow (50 to 1000 mL/min) in line 112 is passed through an H₂O filled container (Humidifier 130) and then is fed into a mixing chamber 140. The helium gas flow in the line 114 is fed directly into the mixing chamber 140. In this manner, with the mass flow controllers 120 on the lines 112, 114 a relative H₂O saturation in the gas exiting the chamber 140 can be adjusted. Adjustment of the gas flow in the two lines 112, 114 makes the overall flow rate and humidity fine tuning of the gas flow exiting the chamber 140 possible. The humidity may be in the range of 20%-100% with a preferred humidity of at least 70%. The total helium flow in this embodiment could be varied from 0.5 L/min to 5 L/min in all cases. In this Helium Gas Humidity Adjustment Setup, the Helium H₂O vapor content was varied during experiments. The humidity of Helium gas in the chamber 140 was measured via calibrated High-Accuracy Humidity and Temperature Meter 142 as shown in the FIG. 7A. The humidified helium gas from the chamber 140 is fed into a CAP generator 300,

At the same time, an un-humidified air tank 150 and un-humidified oxygen tank 160 feed air and oxygen respectively to a respiratory delivery system 170, such as a ventilator, CPAP (continuous positive airway pressure) system, or BIPAP (Bilevel Positive Airway Pressure) system. These are referred to herein as "feed gases." The respiratory delivery system 170 will mix, adjust, and measure the pressure, flow rate, ratio, and frequency of the patient inbreath of air, oxygen, and CO₂. The output of the CAP generator 300 and the respiratory delivery system 170 are connected to a dielectric barrier discharge (DBD) assembly 200. The output of the CAP joint mixer 200 is connected to a delivery member 190, which, for example, may be an endobronchial tube, oxygen CPAP (continuous positive airway pressure), BIPAP (Bilevel Positive Airway Pressure), ventilator face mask, or nasal O₂ cannula 190 to deliver reactive species 192, e.g., H2O2, NO2-, NO3-, ONOO-, and 02-, generated by the system into the patient's respiratory system.

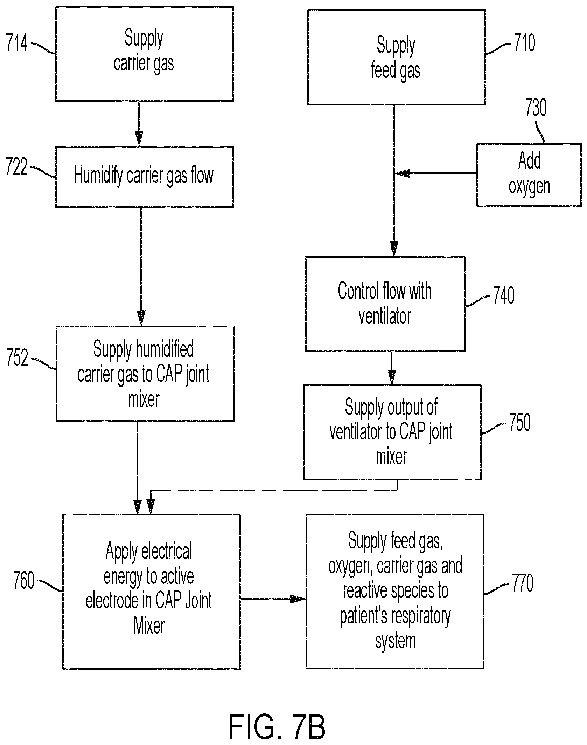

A method for treating a respiratory infection with the system of FIG. 7A is described with reference to FIG. 7B. A CAP carrier gas such as helium is supplied to a humidifier 714. The carrier gas is humidified 722. The humidified carrier gas is supplied to a CAP generator. At the same time, pressurized feed gas (air) is supplied 710. Oxygen is added to the air flow 730. The air and oxygen flow is controlled by a ventilator or other respiratory delivery system 740. The humidified CAP carrier gas from the CAP generator and the output of the ventilator both are supplied to a CAP joint mixer 750, 752. Electrical energy is applied to an inner electrode in the CAP joint mixer 760. The output of the CAP joint mixer is then supplied to the patient's respiratory system 770, for example, via a respiratory face mask, nasal cannula, or endobronchial tube.

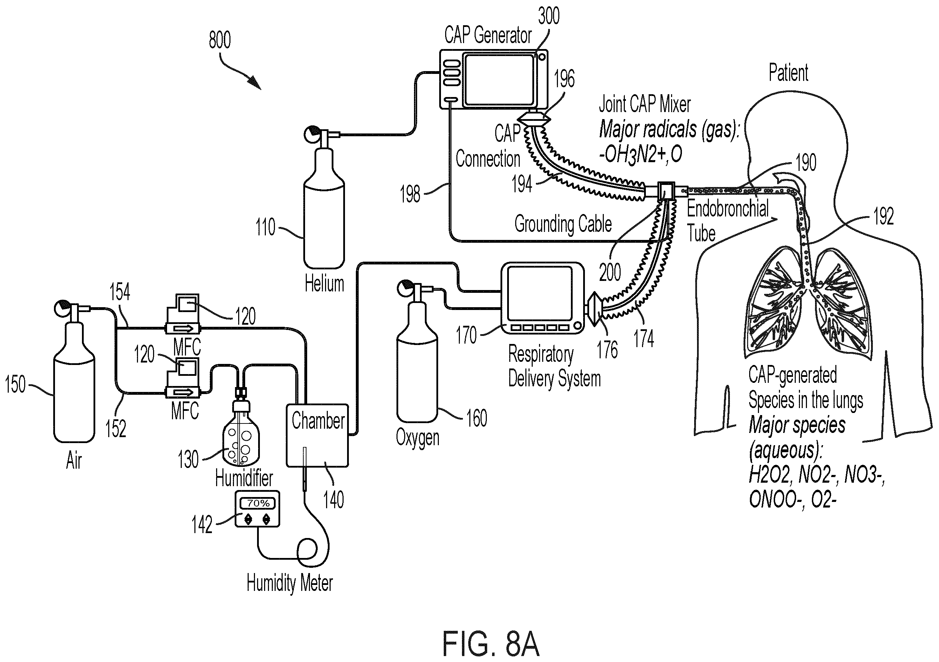

Another embodiment of a cold atmospheric plasma system 800 for treatment of respiratory infections is described with reference to FIG. 8A. A helium gas source 110 is fed into an electrosurgical generator 300. At the same time, an un-humidified air supply 150 (feed gas) is split into two lines 152, 154. Each line 152, 154 is controlled by a mass flow controllers (MFC) 120. The air gas flow in line 152 is passed through an H₂O filled container (Humidifier 130) and then is fed into a mixing chamber 140. The air gas flow in the line 114 is fed directly into the mixing chamber 140. In this manner, with the mass flow controllers 120 on the lines 152, 154 a relative H₂O saturation in the air feed exiting the chamber 140 can be adjusted. Adjustment of the air flow in the two lines 152, 154 makes the overall flow rate and humidity fine tuning possible. The humidity may be in the range of 20%-100% with a preferred humidity of at least 70%. The humidity of air in the chamber 140 is measured via calibrated High-Accuracy Humidity and Temperature Meter 142. The humidified air from the chamber 140 and oxygen from an oxygen supply 160 are provided to a respiratory delivery system 170, such as a ventilator, CPAP machine, BIPAP machine, or other known respiratory deliver system. The respiratory delivery system 170 will mix, adjust and measure the pressure, flowrate, ratio and frequency of the patient inbreath of exhaust air, oxygen and CO2.

The output of the CAP joint mixer 200 is connected to a delivery member 190, which, for example, may be an endobronchial tube, oxygen CPAP (continuous positive airway pressure), BIPAP (Bilevel Positive Airway Pressure), ventilator face mask, or nasal O₂ cannula 190 to deliver reactive species 192, e.g., H2O2, NO2-, NO3-, ONOO-, and 02-, generated by the system into the patient's respiratory system.

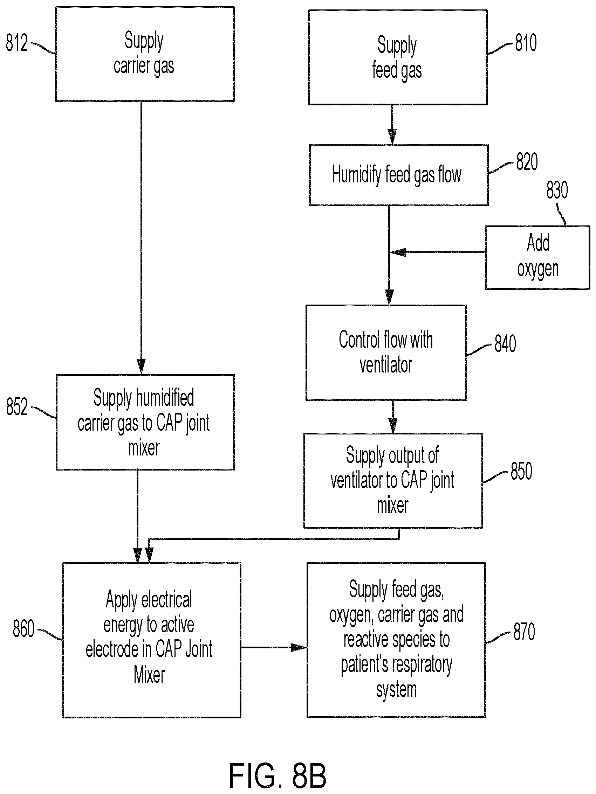

A method for treatment a respiratory infection in which the feed gas (air) is humidified is described with reference to FIG. 8B. Pressurized feed gas (air) is supplied to a humidifier 810. The pressurized air is humidified in a humidifier 820. Oxygen is added to the humidified air flow 830. The humidified air and oxygen flow is controlled by a ventilator or other respiratory delivery system 840. At the same time, a CAP carrier gas such as helium is supplied to a CAP generator 812. The CAP carrier gas from the CAP generator and the output of the ventilator both are supplied to a CAP joint mixer 850, 852. Electrical energy is applied to an inner electrode in the CAP joint mixer 860. The output of the CAP joint mixer is then supplied to the patient's respiratory system 870, for example, via a respiratory face mask, nasal cannula, or endobronchial tube. Other embodiments of the invention are possible in which the plasma is delivered to a patient, for example, through an endoscopic or laparoscopic device. Still further, in other embodiments, the present invention may treat cancer in the abdomen by feeding the output of the CAP joint mixer into the abdomen, for example, via a laparoscope or trocar.

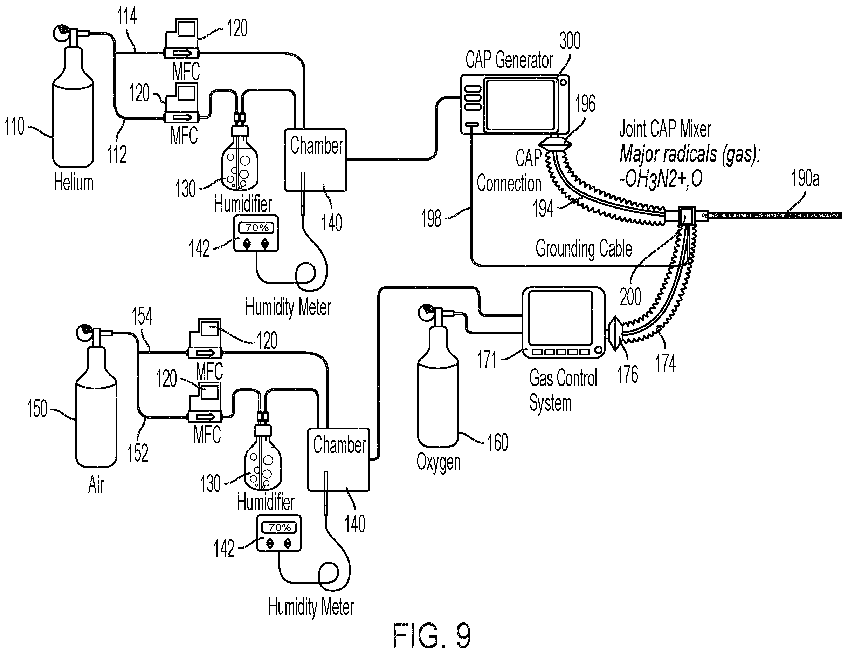

A cold atmospheric plasma system for treatment of a patient via an endoscope or laparoscope in accordance with a preferred embodiment of the present invention is described with reference to FIG. 9. A helium gas source 110 is split into two lines 112, 114, with each of the two lines controlled by a mass flow controller (MFC) 120. The Helium gas flow (50 to 1000 mL/min) in line 112 is passed through an H₂O filled container (Humidifier 130) and then fed into a mixing chamber 140. The helium gas flow in the line 114 is fed directly into the mixing chamber 140. In this manner, with the mass flow controllers 120 on the lines 112, 114 a relative H₂O saturation in the gas exiting the chamber 140 can be adjusted. Adjustment of the gas flow in the two lines 112, 114 makes the overall flow rate and humidity fine tuning of the gas flow exiting the chamber 140 possible. The humidity may be in the range of 20%-100% with a preferred humidity of at least 70%. The total helium flow in this embodiment could be varied from 0.5 L/min to 5 L/min in all cases. The humidity of Helium gas in the chamber 140 is measured via calibrated High-Accuracy Humidity and Temperature Meter 142. The humidified helium gas from the chamber 140 is fed into an electrosurgical generator 300. A variety of electrosurgical generators are known in the art and could be used with the present invention. The gas being fed into the Cold Atmospheric Plasma (CAP) Generator 300 is referred to herein as the "carrier gas."

At the same time, an un-humidified air supply 150 (feed gas) is split into two lines 152, 154. Each line 152, 154 is controlled by a mass flow controllers (MFC) 120. The air gas flow in line 152 is passed through an H₂O filled container (Humidifier 130) and then is fed into a mixing chamber 140. The air gas flow in the line 154 is fed directly into the mixing chamber 140. In this manner, with the mass flow controllers 120 on the lines 152, 154 a relative H₂O saturation in the air feed exiting the chamber 140 can be adjusted. Adjustment of the air flow in the two lines 152, 154 makes the overall flow rate and humidity fine tuning possible. The humidity may be in the range of 20%-100% with a preferred humidity of at least 70%. The humidity of air in the chamber 140 is measured via calibrated High-Accuracy Humidity and Temperature Meter 142. Humidified air from the chamber 140 and oxygen from an oxygen supply 160 are connected to a gas control system 171. In an alternative embodiment, an integrated gas-enhanced electrosurgical generator having a plurality of gas control modules 1000a, 1000b, 1000c such as is shown in FIG. 3D may be used. In such a system the flow helium, air and oxygen all are controlled by gas modules in a single housing and having a unified control system.

The output of the CAP generator 300 and humidified air and oxygen from the gas control system 170 are connected to a dielectric barrier discharge (DBD) assembly 200, referred to herein as a "CAP joint mixer." A ground cable 198 connects an outer electrode of the CAP joint mixer 200 to a ground in the CAP generator 200. While the grounding cable 198 is shown separate from the tubing 194 in FIG. 1, other arrangements are possible in which the ground cable 198 is combined, for example, in a harness with the tubing 194. Due to the presence of the H₂O, the ionization of Helium and H₂O to He⁺ + e⁻ chemical reaction will happen simultaneously. The cold plasma-generated reactive species (H2O2, NO2-, NO3-, ONOO-, and 02-) are produced.

The output of the CAP joint mixer 200 is connected to an elongated delivery member 190a, which, for example, may be a rigid or flexible tube of a size that will fit into a channel of any type of endoscope or laparoscope, whether the scope is a bronchoscope, colonoscope or any other type of scope used in surgical applications.

The embodiment shown in FIG. 1A where both the feed gas (air) and the carrier gas (helium) are humidified provides greater humidity in CAP joint mixer than embodiments where only one of the feed gas and carrier gas is humidified. In the embodiment of FIG. 1A, experiments have shown that due to the increased humidity, the treatment time necessary to achieve a 100% kill rate for lung cancer cells can be reduced to 5 minutes versus about 17 minutes for the embodiments where only one of the feed gas and carrier gas is humidified. Further, the production of ozone can be reduced from roughly 20 ppm (parts per million) for the embodiments of FIGs. 7A and 8A to less than 3 ppm (approximately 2 ppm) in the embodiment of FIG. 1A.

A gas control module 1000 in accordance with the present invention is designed for gas-enhanced electrosurgical systems. Conventionally, gas-enhanced electrosurgical systems have an electrosurgical generator and a gas control unit that have separate housings. The conventional gas control unit typically controls only a single gas such as argon, CO₂ or helium. The present invention uses a gas control module 1000 that may be used in a gas control unit or in a combined unit functioning both as an electrosurgical generator and as a gas control unit. Further, a plurality of gas control modules in accordance with the present invention may be combined in a single gas control unit or combination generator/gas control unit to provide control of multiple gases and provide control for multiple types of gas-enhanced surgery such as argon gas coagulation, hybrid plasma electrosurgical systems and cold atmospheric plasma systems.

Still further, while helium is the carrier gas used in the disclosed embodiments, other gases such as argon, nitrogen, oxygen or air may be used as a carrier gas.

While the preferred embodiments are described with a ventilator, other medical respiration devices such as a continuous positive airway pressure (CPAP) system could be used with the present invention.

### Experiments

The cold atmospheric plasma system for treatment of respiratory infections where only the air was humidified was used to treat 1 mL phosphate buffer saline (PBS) in 12-well plates with the generator in Argon Coag Mode and Spray Mode operating at a frequency near 100kHz for 3 min each. Voltage was set to be 70 V. Oxygen and air flow rate were both set to be 1 LPM. The mixture of oxygen (O₂) and air was humidified by bubbling through DI water. The relative humidity (RH) of the mixture is about 80%. Flow rate of helium, the carrier gas for cold atmospheric plasma (CAP), was set at 3 LPM. Therefore, the O₂ percentage of the final output gas from the endobronchial tube was about 24% in the O₂-air-He mixture.

Among of the cocktail of plasma-generated reactive oxygen species (ROS) and reactive nitrogen species (RNS) in the treated solution, hydrogen peroxide (H₂O₂) and nitrite (NO₂⁻) are the most commonly studied long-lived species. Their concentrations were measured in treated phosphate-buffered saline (PBS) using Griess Reagent System (Promega, G2930) and colorimetric Hydrogen Peroxide Assay Kit (Sigma-Aldrich, MAK311-1KT) with CAP on or off. Results were read by a BioTek microplate reader at 550 nm and 595 nm for absorbance, respectively.

Electrosurgical generators typically have multiple modes of operation, including "cut" or cutting modes and "coag" or coagulation modes of operation. A cut mode typically will have a low voltage waveform form (e.g., 1KV) with a high duty cycle, e.g. 100%. The coag mode of an electrosurgical generator typically creates a waveform with large amplitude but short duration "spikes" to achieve hemostasis (coagulation). For example, a coag mode on an electrosurgical generator may use a high voltage wave form at a 6% duty cycle. Different degrees of hemostasis (coagulation) can be achieved by utilizing varying degrees of "Blended" waveforms, e.g., 50% on/50% off, 40% on/60% off, or 25% on/75% off. Electrosurgical generators also have argon plasma coagulation modes, or "argon coag" modes. Argon Plasma Coagulation (APC) utilizes plasma produced by the ionization of a few millimeter diameter argon flow exhausting into ambient air from the electrosurgical hand-piece. When compared to a cut mode, an argon coagulation mode on a generator may use a high voltage (e.g. ,4 KV for argon coag versus 1KV for a cut mode), less current (e.g., 200mA for argon coag versus 500mA for cut), and lower frequency (30KHz for argon coag versus 390 KHz for cut). Electrosurgical generators also have a "Spray Mode," which is similar to the argon coag mode (similar voltage and current), but they have a random week of frequency, for example, from 10-30 KHz, which allows it to cover different tissue impedances.

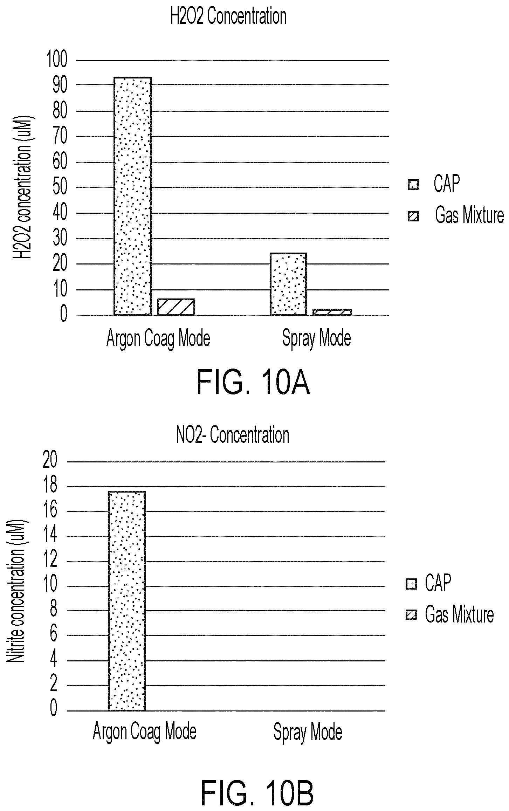

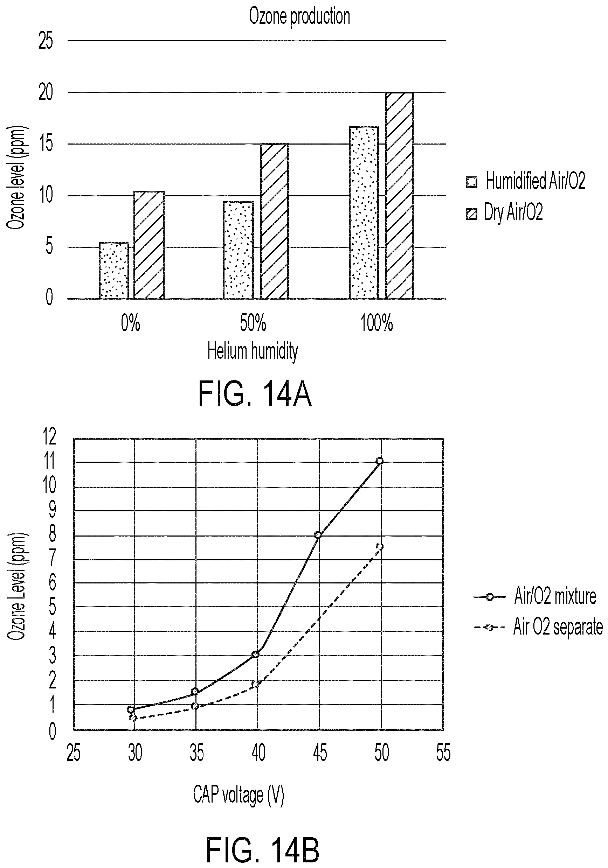

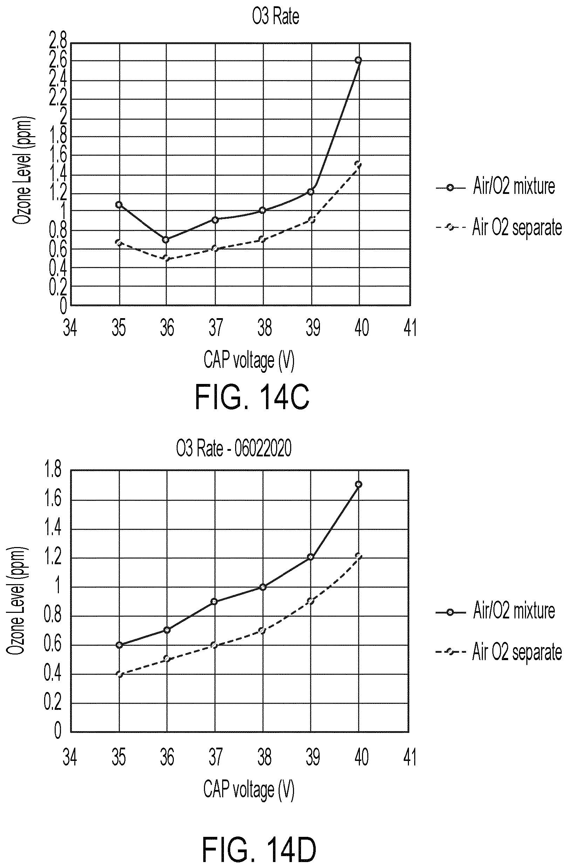

The concentrations of H₂O₂ and NO₂⁻ with the present system treatment were plotted in FIGs. 14A and 14B. With CAP turned on, both species are higher when treated in Argon Coag Mode than when treated in Spray Mode. Gas-only treatment was also performed as a control. As indicated in FIGs. 10A and 10B, with 3 min of treatment, Argon Coag Mode at 70 V generated 90 µM H₂O₂ and 18 µM NO₂⁻; whereas Spray Mode at 70 V generated 25µM H₂O₂ and undetectable amount of NO₂⁻. A gas mixture alone does not generate significant amount of ROS or RNS.

### CAP Plasma Ventilator Validation

Cold atmospheric plasma has been reported to induce inactivation of airborne viruses (Xia, T., et al., Inactivation of airborne viruses using a packed bed non-thermal plasma reactor. Journal of Physics D: Applied Physics, 2019. 52(25)), deactivation of hepatitis B virus while keeping normal liver function during CAP treatment (Shi, X.-M., et al., Effect of Low-Temperature Plasma on Deactivation of Hepatitis B Virus. IEEE Transactions on Plasma Science, 2012. 40(10): p. 2711-2716), inhibition of HIV replication (Volotskova, O., et al., Cold Atmospheric Plasma Inhibits HIV-1 Replication in Macrophages by Targeting Both the Virus and the Cells. PLoS One, 2016. 11(10): p. e0165322), inactivation of Newcastle disease virus and avian influenza virus without destruction of the antigenic determinants for vaccine preparation (Wang, G., et al., Non-thermal plasma for inactivated-vaccine preparation. Vaccine, 2016. 34(8): p. 1126-32) and so forth. In this study, CAP is combined with a ventilator system to achieve the delivery of CAP as well as the treatment of the virus throughout the patient's respiratory system.

Wu el al (Wu, Y., et al., MS2 virus inactivation by atmospheric-pressure cold plasma using different gas carriers and power levels. Appl Environ Microbiol, 2015. 81(3): p. 996-1002) suggested that the ambient air as carrier gas produced the highest level of inactivation at power levels of 20 and 24 W, followed by the gas carriers Ar-O₂ (2%, vol/vol) and He-O2 (2%, vol/vol). In addition, air is a required input gas for all ventilators. Hence air as carrier gas is the best option for a CAP-equipped ventilator. Relative humidity (RH) as an important factor of the air will be studied for an optimal configuration in addition to CAP treatment parameters including discharge voltage (V) and treatment time (t).

### CAP-generated reactive species

Reactive species generated by CAP can be confirmed within the plasma beam using optical emission spectroscopy (OES) and in aqueous solution by kits based on species.

### Reactive species in the plasma beam

An optical emission spectrometer (Ocean Optics HR2000) is used to detect the species in the plasma beam in the range of 200-900 nm. The plasma emissions are collected in a direction perpendicular to the plasma beam axis and at 1-mm increments in axial direction using a collimating lens. The plasma emission is transmitted to the spectrometer via optical fiber.

### Reactive species in the solution

Kondeti et al did a thorough research on the species generated in CAP-treated saline and water based on their half-lives. Kondeti, V., et al., Long-lived and short-lived reactive species produced by a cold atmospheric pressure plasma jet for the inactivation of Pseudomonas aeruginosa and Staphylococcus aureus. Free Radic Biol Med, 2018. 124: p. 275-287. They concluded that long-lived species played a dominant role when the plasma was not in direct contact with the saline; whereas short-lived species was more important when the plasma touched the liquid. Long-lived species in CAP-treated solution like NO₂⁻ and H₂O₂ concentrations can be measured in air flow-treated phosphate buffer saline (PBS) using Griess Reagent System (Promega, G2930) and Fluorimetric Hydrogen Peroxide Assay Kit (Sigma-Aldrich, MAK165-1KT) with CAP on or off. Results will be read by a BioTek microplate reader at 540 nm for absorbance and 540/590 nm for fluorescence, respectively.

Ozone can be a concern for CAP-based ventilator because it can have detrimental impacts on human health. Ozone concentration should be measured at the exhaust of the ventilator and reduced with filters to meet the air quality standards.

### CAP effect on the cells

Lung cancer cell line A549 will be used for the efficacy of CAP treatment. Air flow-only treatment will be used as control group. The viability of the cells will be evaluated by 3-(4,5-dimethylthiazol-2-yl)-2,5-diphenyl tetrazolium bromide (MTT) assay.

In conclusion, with tuned configuration, CAP-based ventilators could benefit patients with respiratory diseases like pneumonia, COVID19, or lung cancer.

### CAP Plasma Virus Inactivation Validation

Respiratory disease-causing viruses such as COVID-19 and severe acute respiratory syndrome (SARS) are transmitted by aerosolized droplets containing the infection virus. Cold atmospheric-pressure plasma (CAP) generates numerous reactive oxygen species (ROS) and reactive nitrogen species (RNS), such as hydrogen peroxide (H₂O₂), singlet oxygen (¹O2), ozone (O3), nitric oxide (^{·}NO), and hydroxyl radical (^{·}OH), as well as electrons, ions, and photons, in which ^{·}OH, ¹O₂, ^{·}NO, O₂^{·}-, ^{·}NO₂, and ONOO- are short-lived species whereas H₂O₂, NO₂-, and NO₃-, are long-lived species. Various studies have shown that the CAP could inactivate viruses and other microbes. The potential inactivation mechanisms of the virus by the CAP is by inducing to high oxidation-reduction potential (ORP) and electrical conductivity by producing large number of free radicals. The reactive oxygen and nitrogen species could react with carbohydrates and initiate lipid peroxidation and cross-linking of the fatty acid side chains, resulting in alterations of the chemical bonds and molecular structure. They induce oxidative stress by causing protein peroxidation and inducing the destruction of the virus envelope, singlet oxygen could rapidly react with cysteine to generate the major product of cystine (R-cys-S-S-cys-R) with disulfides, they selectively reacted with tyrosine, tryptophan, and histidine to produce hydroperoxides resulting in protein aggregation and ultimately resulting in changes to the viral morphology. Moreover, they can damage viral nucleic acids encoding enzymes, by oxidizing guanine and induce cross-links between guanine and lysine contributing to reduced gene expression and the elimination of virus replication, thereby leading to virus inactivation. The cold plasma system of the present invention generates ionized cold plasma in a humidified setup to produce reactive species that are fed to virus infected patient via endobronchial tube. The output of the system contains reactive oxygen species (ROS) and reactive nitrogen species (RNS) which would inactivate the virus present in the patient's bronchial cells.

Human epithelial lung carcinoma cell line A549 (ATCC, CCL-185) was used to study the efficacy of the present invention. A549 cells were seeded at a density of 10⁵/well in 12-well plates and treated for up to 17 minutes. The frequency was near 100 kHz. Voltage was set to be 70 V. Flow rate of helium, the carrier gas for cold atmospheric plasma (CAP), was set at 3 LPM. The total flow rate of oxygen (O₂) and air were set to be 2 LPM. The O₂ percentage of the final output gas from the endotracheal tube was about 16, 24, and 32% in the O₂-air-He mixture.

The feed gas of O₂ and air mixture or He was humidified by bubbling through DI water. The relative humidity (RH) of the humidified gas was measured constantly with a humidity sensor.

Thiazolyl blue tetrazolium bromide (MTT) was purchased from Sigma-Aldrich (St. Louis, MO, USA) and viability assays were carried out 48 hours after CAP treatment according to the manufacturer's protocol. Results were read by a BioTek microplate reader at 570 nm for absorbance.

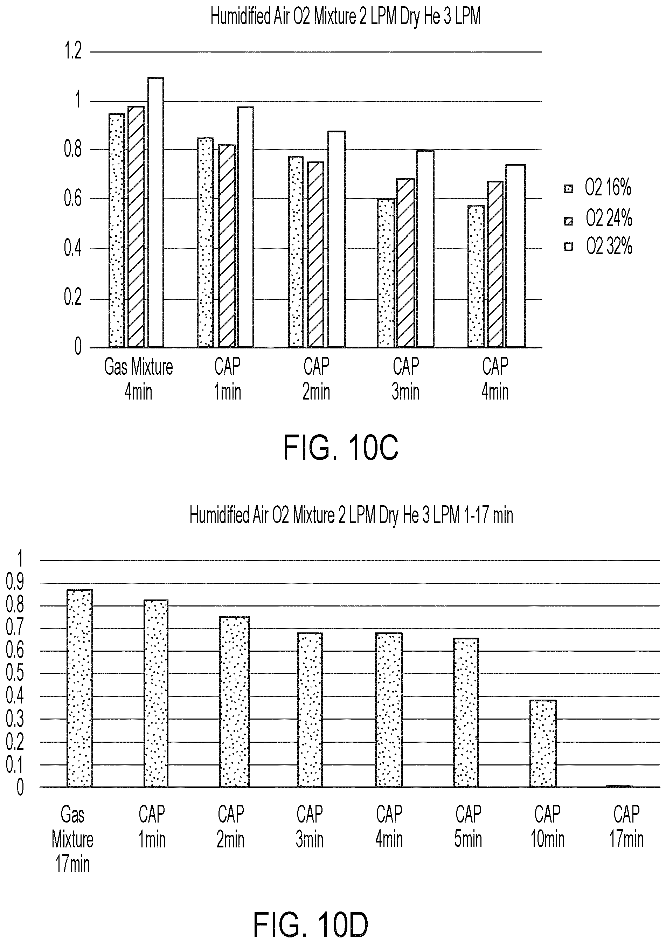

The viability of A549 cells treated by the present invention were plotted, as shown in FIGs. 10C-10E. FIG. 10C shows the viability of A549 cells treated by the setup of the present invention with air mixture humidity adjustment for up to 4 minutes. The viability of A549 cells was decreased gradually with increasing treatment time. With 4 min of treatment, the viability was reduced to 60% (compared to no treatment). Oxygen fraction increasing in the gas mixture has indicated a weakened effect of the treatment from the FIG. 10C.

The same setup with 24% O₂ in the feeding gas was then used to treat the cells for up to 17 min (FIG. 10D). Cell viability was reduced below 40% after 10 min of treatment compared to no treatment, and the cells were completely eliminated after 17 min of treatment.

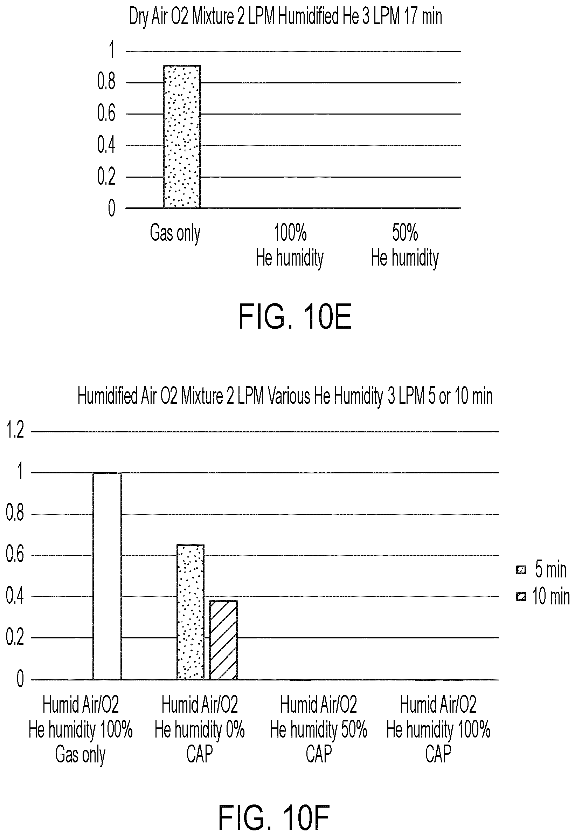

When treated with the setup where only the helium is humidified, with helium humidity adjustment for 17 min, all the cancer cells were eliminated by the CAP treatment as well (data shown in FIG. 10E).

### Effect of Treatment with humidified Air/O2 mixture and humidified Helium

A549 cells were treated with 100% humidified Air/O₂ mixture (1:1 v/v) at a flow rate of 2 LPM (O₂ fraction 24%). Helium flow rate was 3 LPM and humidity was set at 0%, 50%, and 100%.

A549 cells without treatment attached firmly to the culture dish, and nuclei were intact. The control in this experiment was Air/O₂ and He mixture-treated for 10 min. Cells did not demonstrate any morphological changes compared to no treatment.

When He humidity was set at 0% (dry helium), the cells started to shrink within 5 min of cold plasma system treatment, but a significant amount of the cells were still viable. After increasing the treatment time to 10 min, cell death was identified.

When He humidity was increased to 50%, at 5 min of treatment time, the cells demonstrated shrinkage and blebbing of the membrane. Cell shrinkage was more severe at 10 min treatment time, and dead cells were visualized in a floating pattern.

When He humidity was increased to 100% at 5 or 10 min of treatment, almost all the cells were fragmented and not viable.

An MTT viability assay was performed on the cells. The results are shown in FIG. 10F. He humidity at 0% (dry helium) and 5min treatment time reduced the viability to 60%, and at 10 min the viability was reduced the viability to 40% compare to no treatment. When He humidity was set to 50% or 100%, there were no viable cells at 5 or 10 min of treatment.

A more comprehensive study was performed to determine the minimum treatment time required for elimination of A549 cells. A549 cells were treated for 1-5 min with humidified or dry Air/O₂ mixture (1:1 v/v) at a flow rate of 2 LPM (O₂ fraction 24%). Helium flow rate was 3 LPM and humidity was set at 0%, 50%, and 100%. Images were taken 24 hour-post CAP treatment.

Phase contrast images of the A549 cells treated with a system in accordance with a preferred embodiment of the present invention for 1 - 5 min with humidified or dry Air/O₂ mixture and various humidity of He were taken. When He humidity was set at 0% (dry helium), cell number started to decrease at 5 min of treatment, but cell morphology did not change significantly; when He humidity was set at 50%, cell number started to decrease at 4 min of treatment; when He humidity was set at 100%, cell number started to decrease at 2 min of treatment, and cell membrane and nuclei started to shrink significantly at 4 min of treatment. Humidity of Air/O₂ did not induce significant morphological changes.

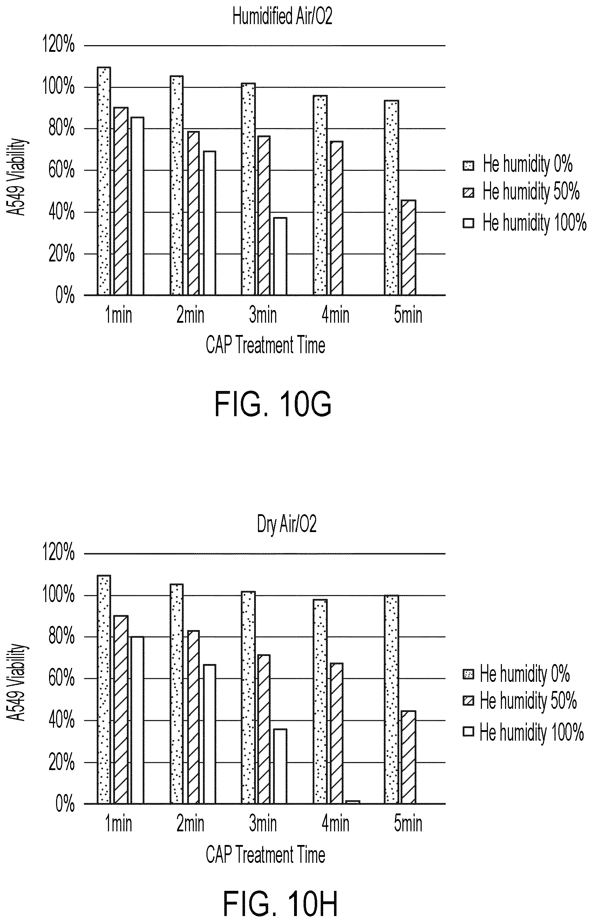

An MTT viability assay was performed on the cells (FIGs. 10G and 10H). He humidity at 0% (dry helium) did not induce much cell death compared to no treatment even at 5 min of treatment. When He humidity was set to 50%, cell viability gradually decreased with increasing of treatment time. About 50% of cells were viable at 5 min of treatment. When He humidity was set to 100%, 3 min of CAP treatment was able to reduce viability to below 50%, and 4 min of treatment completely eliminated the cells. Humidity of Air/O₂ did not result in significant differences in viability data. Based on these results, one may conclude that humidification of helium is a critical factor for the cold plasma system to eradicate lung cancer cells.

### Separating Oxygen Flow from Air Flow

A cold atmospheric plasma system for treatment of respiratory infections in accordance with a first preferred embodiment of the present invention is described with reference to FIG. 11. A helium gas source 1110 is split into two lines 1112, 1114, with each of the two lines controlled by a mass flow controller (MFC) 1120. The Helium gas flow (50 to 1000 mL/min) in line 1112 is passed through an H₂O filled container (Humidifier 1130a) and then fed into a mixing chamber 1140a. The helium gas flow in the line 1114 is fed directly into the mixing chamber 1140a. In this manner, with the mass flow controllers 1120 on the lines 1112, 1114 a relative H₂O saturation in the gas exiting the chamber 1140a can be adjusted. Adjustment of the gas flow in the two lines 1112, 1114 makes the overall flow rate and humidity fine tuning of the gas flow exiting the chamber 1140a possible. The humidity may be in the range of 20%-100% with a preferred humidity of at least 70%. The total helium flow in this embodiment could be varied from 0.5 L/min to 5 L/min in all cases. The humidity of Helium gas in the chamber 1140a may be measured, for example, via calibrated High-Accuracy Humidity and Temperature Meter (not shown). The humidified helium gas from the chamber 1140a is fed into an electrosurgical generator 300, referred to herein as a "Cold Atmospheric Plasma (CAP) Generator." A variety of electrosurgical generators are known in the art and could be used with the present invention. The gas being fed into the Cold Atmospheric Plasma (CAP) Generator 300 is referred to herein as the "carrier gas."

At the same time, an un-humidified air supply 1150 (feed gas) is controlled by a mass flow controller (MFC) 1120. The air gas flow is passed through a second H₂O filled container (Humidifier 1130b) and then is fed into a mixing chamber 1140b. Also at the same time, a source 1160 of an unhumidified pressurized third gas, oxygen in this case, is connected to a third H₂O filled container (Humidifier 1130c). The humidified third gas (oxygen) is fed into chamber 1140b where it mixes with the humidified air. In this manner, with the mass flow controllers 1120 on the air and oxygen lines the relative oxygen percentage exiting the chamber 1140b can be adjusted. The humidity of each of the air and oxygen may be in the range of 20%-100% with a preferred humidity of at least 70%. The humidity of mixture in the chamber 1140b may be measured, for example, via a calibrated High-Accuracy Humidity and Temperature Meter (not shown) and the oxygen content may be measured for example with an oxygen sensor. The humidified air and oxygen from the chamber 1140b are provided to a respiratory delivery system 1170, such as a ventilator, CPAP machine, BIPAP machine, or other known respiratory deliver system. The respiratory delivery system 1170 will mix, adjust and measure the pressure, flowrate, ratio and frequency of the patient inbreath of exhaust air, oxygen and CO2. The output of the respiratory delivery system 1170 is connected to the CAP joint mixer, for example, via tubing 1174 and connector 1176.

The output of the CAP generator 300 and the respiratory delivery system 1170 are connected to a dielectric barrier discharge (DBD) assembly 200, referred to herein as a "CAP joint mixer." A ground cable 1198 connects an outer electrode of the CAP joint mixer 200 to a ground in the CAP generator 300. While the grounding cable 1198 is shown separate from the tubing 1194 in FIG. 11, other arrangements are possible in which the ground cable 1198 is combined, for example, in a harness with the tubing 1194. Due to the presence of the H₂O, the ionization of Helium and H₂O to He⁺ + e⁻ chemical reaction will happen simultaneously. The cold plasma-generated reactive species (H2O2, NO2-, NO3-, ONOO-, and 02-) are produced.

The output of the CAP joint mixer 200 is connected to a delivery member 1190, which, for example, may be an endobronchial tube, oxygen CPAP (continuous positive airway pressure), BIPAP (Bilevel Positive Airway Pressure), ventilator face mask, or nasal O₂ cannula 1190 to deliver reactive species 1192, e.g., H2O2, NO2-, NO3-, ONOO-, and 02-, generated by the system into the patient's respiratory system.

### Ozone Measurements

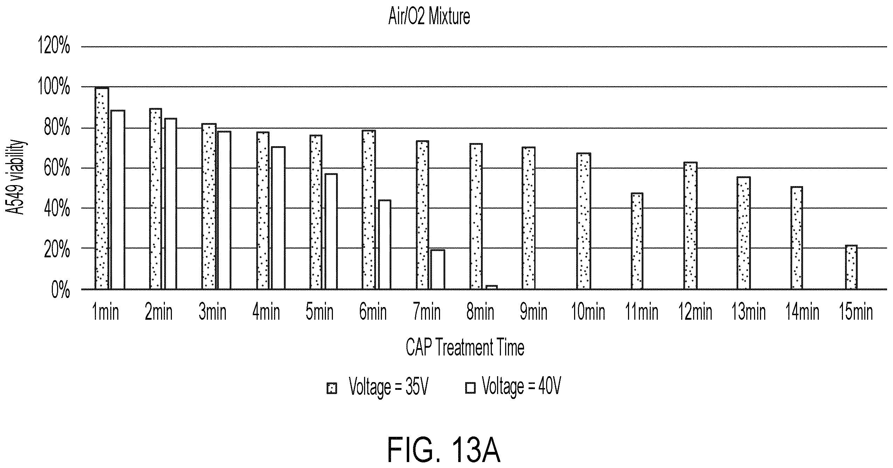

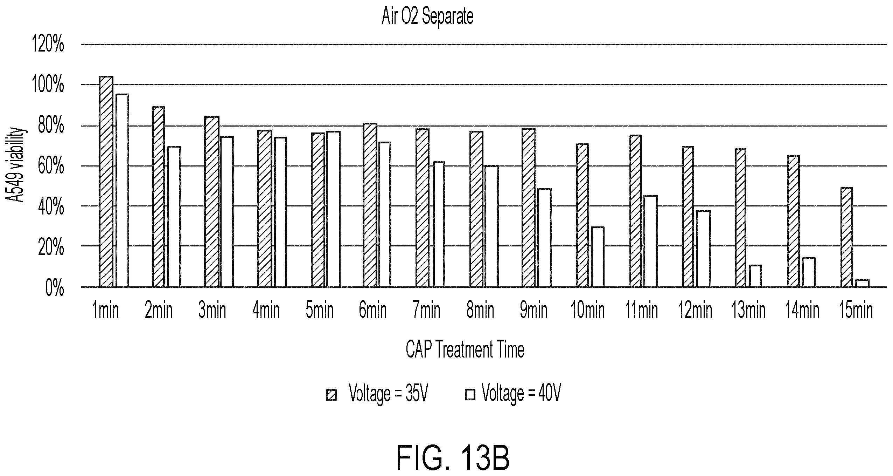

Ozone (O₃) generated by a system in accordance with a preferred embodiment of the present invention was measured at the end of the endotracheal tube with an ozone detector (Forensics Detectors, CA). The measurement was carried out with all settings tested above, *i.e.,* CAP was set to 70 V with humidified or dry Air/O₂ mixture (1:1 v/v) at a flow rate of 2 LPM (O₂ fraction 24%) and helium flow rate was 3 LPM and humidity was set at 0%, 50%, and 100%. Ozone level was shown in FIG. 14A. At the same helium humidity, dry Air/O₂ yielded higher O₃ level compared to humidified Air/O₂. Higher humidity of helium generated higher concentration of O₃, which resulted in stronger reduction effect on the cells as shown earlier in FIGs. 10G and 10H. This correspondence indicates that O₃ is a critical species in the cocktail that generated by a system in accordance with a preferred embodiment of the present invention. FIG. 14B shows ozone production rate significantly decreased at lower voltage. Therefore, 35 or 40 V was used to test cell viability instead of 70 V for safety purpose (FIGs. 13A and 13B). O₃ production rate was higher when Air and O₂ were fed into the system as a mixture (FIG. 14B) compared to separate Air and O₂ infusion (FIG. 14B).

However, the cold plasma system with previously demonstrated settings, i.e., CAP was set to 70 V with humidified or dry Air/O₂ mixture (1:1 v/v) at a flow rate of 2 LPM (O₂ fraction 24%) and helium flow rate was 3 LPM and humidity was set at 0%, 50%, and 100%, produced a large amount of ozone (data demonstrated in Section 2) which is over the safety limit by OSHA standard. In order to lower the ozone generation, lower voltage (35 - 40 V) was utilized to treat the cells. Because the presence of oxygen in the Air/O₂ mixture inflamed the ozone production, Air and O₂ were fused into the joint CAP mixer separately to lower the O₃ formation. The viability data of A549 cells treated at settings with low O₃ level (i.e. lower voltage and separation of Air and O₂) is shown in FIGs. 14A and 14B.

When Air and O₂ were added to the system as a mixture (FIG. 13A), the capability of CAP on reducing cancer viability was higher compared to where Air and O₂ were fused to the system separately (FIG. 13B). CAP treatment of 8 min at 40 V with Air/O₂ mixture or 15 min at 40 V with Air and O₂ separation were able to lower cancer cell viability to less than 5% percent.

### Reactive Species Detection in the Treated Medium

A system in accordance with a preferred embodiment of the present invention was used to treat 1 mL Phosphate Buffer Saline (PBS) in 12-well plates with Argon Coagulation Mode for 8 or 15min continuously and in intervals. For interval treatment, CAP was administered in 3 + 3 + 2 min or 4 + 4 + 4 + 3 min manner with 5 min break between each interval. Voltage was set at 35 or 40 V. Helium, O₂ and air were all humidified. Flow rate of helium was set at 3 LPM. Oxygen and air flow rate were both set at 1 LPM.

Among the cocktail plasma-generated reactive oxygen species (ROS) and nitrogen species (RNS) in the treated solution, hydrogen peroxide (H₂O₂), nitrite (NO₂⁻) and nitrate (NO₃⁻) are the most commonly studied long-lived species. Their concentrations were measured in treated PBS using colorimetric Hydrogen Peroxide Assay Kit (Sigma-Aldrich, MAK311-1KT), Griess Reagent System (Promega G2930) and colorimetric Nitrite/Nitrate Assay Kit (Sigma-Aldrich 23479), with CAP on or off. Results were read by a BioTek microplate reader at 595 nm, 550 nm, and 540/570 nm for absorbance, respectively.

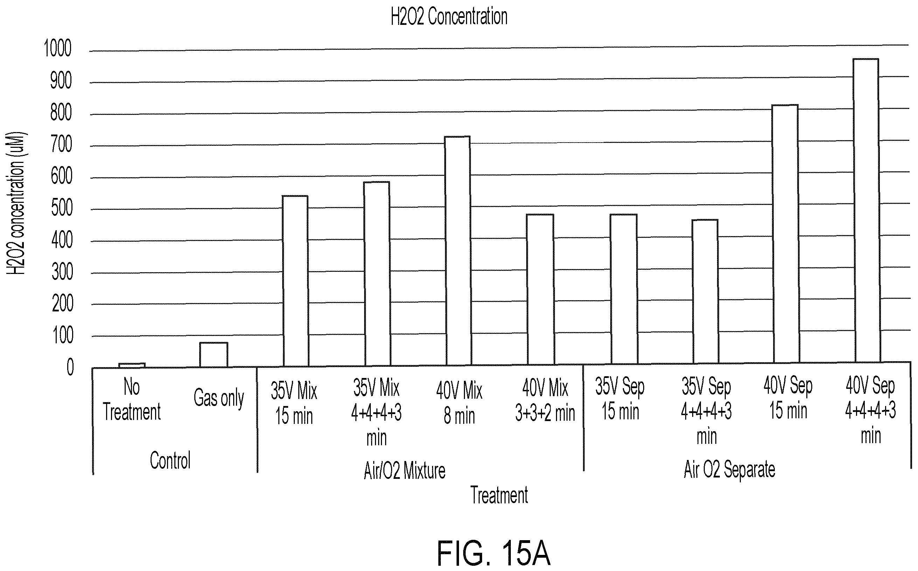

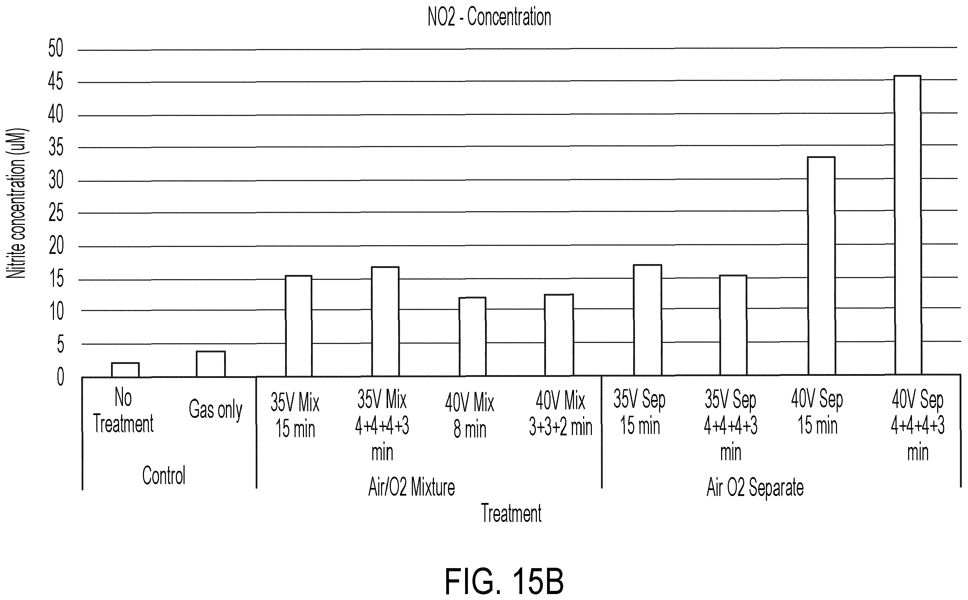

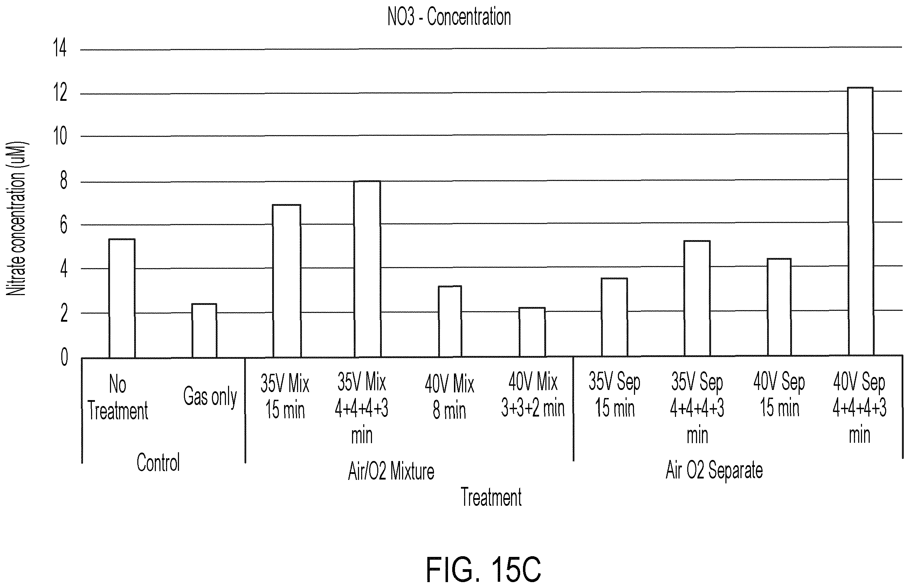

The concentrations of H₂O₂, NO₂⁻ and NO₃⁻with a system in accordance with a preferred embodiment of the present invention were plotted. Previous viability data demonstrated that at 40 V, continuous treatment for 8 min with Air and O₂ mixture setup or 15 min with Air and O₂ separation setup can both lower the viability of A549 to less than 5%. As indicated in FIGs. 15A-15C, 8 min of Air and O₂ mixture setup at 40 V generated 725 µM H₂O₂, 11.9 µM NO₂⁻ and 3.3 µM NO₃⁻ compared to 3 + 3 + 2 min interval treatment generated 470 µM H₂O₂, 12.5 µM NO₂⁻ and 2.2 µM NO₃⁻, whereas continuous 15 min of Air and O₂ separation setup at 40 V generated 806 µM H₂O₂, 33 µM NO₂⁻ and 4.3 µM NO₃⁻ compared to 4 + 4 + 4 + 3 min interval treatment generated 952 µM H₂O₂, 45 µM NO₂⁻ and 12 µM NO₃⁻. Nitrate (NO₃⁻) were too low to detect in most of the settings. Gas mixture alone does not generate significant amount of ROS or RNS.

In the case of 8 min continuous treatment, H₂O₂ was generated in 1 mL of media by CAP treatment with 5 LPM of gas flow. The detected species were as follows:
- 724 × 10⁻⁶ mol/L × 34 g/mol = 24.6 x 10⁻³ mg/mL = 24.6 g/m³ of H₂O₂ was generated by 40 L of gas mixture;
- The H₂O₂ level is 24.6 x 10⁻³ / 40 = 0.615 x 10⁻³ mg/L = 0.615 mg/m³;
- In the case of 3 + 3 +2 min interval treatment, the H₂O₂ level is 0.4 mg/m³;
- In the case of 15 min continuous treatment, the H₂O₂ level is 0.365 mg/m³; and
- In the case of 4 + 4 + 4 + 3 min interval treatment, the H₂O₂ level is 0.43 mg/m³.

In all cases, 0.615, 0.4, 0.38, and 0.42 mg/m³ are lower than NIOSH and OSHA permissible exposure limit for H₂O₂, which is 1.4 mg/m³ (https://www.cdc.gov/niosh/npg/npgd0335.html).

The foregoing description of the preferred embodiment of the invention has been presented for purposes of illustration and description. It is not intended to be exhaustive or to limit the invention to the precise form disclosed, and modifications and variations are possible in light of the above teachings or may be acquired from practice of the invention. The embodiment was chosen and described in order to explain the principles of the invention and its practical application to enable one skilled in the art to utilize the invention in various embodiments as are suited to the particular use contemplated. It is intended that the scope of the invention be defined by the claims appended hereto, and their equivalents.

## Claims

1. A system for performing plasma treatment of respiratory infections comprising: an electrical energy generator (300) configured to generate electrical energy to plasmatize a carrier gas into a plasma; and a dielectric barrier discharge mixer (140) comprising: an interior chamber formed from a dielectric, said interior chamber having a first input (202) configured to fluidly connect to a source of a humidified carrier gas (110), a second input (204) configured to connect to a source of a humidified feed gas (150), and an output (206) configured to connect to a delivery member (190); an active electrode (230) inside said interior chamber and connected to an electrical output of said electrical energy generator; and an outer electrode (220) connected to a ground; wherein a plasma is generated in said interior chamber when electrical energy is supplied from said electrical energy generator (300) to said interior electrode (230) while both humidified feed gas and humidified carrier gas flow into said interior chamber, and further comprising: a first humidifier (130) fluidly connected to said first input (202) of said chamber in said dielectric barrier discharge assembly (140); and a second humidifier (130) fluidly connected to said second input (204) of said chamber in said dielectric barrier discharge assembly (140).

2. A system for performing plasma treatment of respiratory infections according to claim 1, further comprising: a source of a carrier gas (110);
wherein the first humidifier (130) is connected to said source of carrier gas (110); a source of a feed gas (150);
wherein the second humidifier (130) is connected to said source of a feed gas (150); and
a fluid delivery member (190) connected to an output (206) of said mixer (140) for delivering reactive species generated in said mixer (140) to a patient.

3. A system for performing plasma treatment of respiratory infections according to claim 2, wherein said carrier gas comprises at least one of helium, argon, nitrogen and oxygen.

4. A system for performing plasma treatment of respiratory infections according to claim 2, , wherein said delivery member (190) comprises an endobronchial tube.

5. A system for performing plasma treatment of respiratory infections according to claim 2, , wherein said delivery member (190) comprises one of a nasal cannula and a mask.

6. A system for performing plasma treatment according to claim 2, , wherein said source of a feed gas (150) comprises one of a ventilator and a continuous positive airway pressure device.

7. A system for performing plasma treatment of respiratory infections according to claim 6, , wherein said feed gas comprises a mixture of air and oxygen.

8. A system for performing plasma treatment of respiratory infections according to claim 1, wherein said electric energy generator (300) is configured to operate with a frequency in the range of 10kHz to 200kHz and an output peak voltage in the range of 3kV to 6kV.

9. A system for performing plasma treatment according to claim 1, wherein said electric energy generator (300) generates electrical energy having a frequency within 25kHz of one of 40kHz, 100kHz and 200 kHz.

10. A system for performing plasma treatment of respiratory infections according to claim 1, wherein said electric energy generator (300) comprises a high frequency electrosurgical generator and a low frequency converter.

11. A system for performing plasma treatment of respiratory infections according to claim 1, wherein said electric energy generator (300) comprises:
a power module (350);
a CPU (310) for controlling said power module (350);
a memory (311) connected to said CPU (310); and
a power supply (302) connected to said CPU (311).

12. A system for performing plasma treatment of respiratory infections according to claim 11, wherein said electric energy generator (300) further comprises:
a touchscreen display (420);
a controller connected to said touchscreen display; and
a graphical user interface configured to display data on said touchscreen display (420) and receive input from a user through said touch-screen display (420).

13. A system for performing plasma treatment of respiratory infections according to claim 11, wherein said electric energy generator (300) further comprises:
a gas module (1000).

14. A system for performing plasma treatment of respiratory infections according to claim 13, wherein said source of a carrier gas (110) is connected to said gas module (1000) and said gas module (1000) controls a flow of said carrier gas to said mixer (140).

15. A system for performing plasma treatment of respiratory infections according to claim 14, wherein said first humidifier (130) is connected between said gas module (1000) and said mixer (140).

16. A system for performing plasma treatment of respiratory infections according to claim 14, wherein said first humidifier (130) is connected between said gas module (1000) and said source of a carrier gas (110).

17. A system for performing plasma treatment of respiratory infections according to claim 1, wherein said first humidifier (130) is configured to humidify a carrier gas flowing from said source of a carrier gas (110) to at least 70% humidity and said second humidifier (130) is configured to humidify a feed gas flowing from said source of a feed gas (150) to at least 50% humidity.

18. A system for performing plasma treatment of respiratory infections according to claim 1, wherein said first humidifier (130) is configured to humidify a carrier gas flowing from said source of a carrier gas (110) to 100% humidity and said second humidifier (130) is configured to humidify a feed gas flowing from said source of a feed gas (150) to at least 50% humidity.

19. A system for performing plasma treatment of respiratory infections according to claim 1, further comprising:
a source of helium (110) connected to an input of said first humidifier; and
a source of air (150) connected to an input of said second humidifier.

## Patentansprüche

1. Ein System zur Durchführung einer Plasmabehandlung von Atemwegsinfektionen, umfassend:
einen elektrischen Energiegenerator (300), der so konfiguriert ist, dass er elektrische Energie erzeugt, um ein Trägergas in ein Plasma zu plasmatisieren; und einen dielektrischen Barriereentladungsmischer (140), umfassend: eine aus einem Dielektrikum gebildete Innenkammer, wobei die Innenkammer einen ersten Eingang (202), der so konfiguriert ist, dass er mit einer Quelle eines befeuchteten Trägergases (110) fluidisch verbunden ist, einen zweiten Eingang (204), der so konfiguriert ist, dass er mit einer Quelle eines befeuchteten Zufuhrgases (150) verbunden ist, und einen Ausgang (206) aufweist, der so konfiguriert ist, dass er mit einem Abgabemittel (190) verbunden ist; eine aktive Elektrode (230) innerhalb der Innenkammer, die mit einem elektrischen Ausgang des elektrischen Energiegenerators verbunden ist; und eine äußere Elektrode (220), die mit einer Masse verbunden ist; wobei in der Innenkammer ein Plasma erzeugt wird, wenn elektrische Energie von dem elektrischen Energiegenerator (300) zu der inneren Elektrode (230) zugeführt wird, während sowohl befeuchtetes Speisegas als auch befeuchtetes Trägergas in die Innenkammer strömen, und ferner umfassend: einen ersten Befeuchter (130), der fluidmäßig mit dem ersten Eingang (202) der Kammer in der dielektrischen Barriereentladungsanordnung (140) verbunden ist; und einen zweiten Befeuchter (130), der fluidmäßig mit dem zweiten Eingang (204) der Kammer in der dielektrischen Barriereentladungsanordnung (140) verbunden ist.

2. System zur Durchführung einer Plasmabehandlung von Atemwegsinfektionen gemäß Anspruch 1,
das ferner umfasst:
eine Quelle für ein Trägergas (110);
wobei der erste Befeuchter (130) mit der Quelle für das Trägergas (110) verbunden ist;
eine Quelle für ein Zufuhrgas (150);
wobei der zweite Befeuchter (130) mit der Quelle für das Zufuhrgas (150) verbunden ist;
und
ein Fluidzufuhrglied (190), das mit einem Ausgang (206) des Mischers (140) verbunden ist,
um in dem Mischer (140) erzeugte reaktive Spezies einem Patienten zuzuführen.

3. System zur Durchführung einer Plasmabehandlung von Atemwegsinfektionen gemäß Anspruch 2,
wobei das Trägergas mindestens eines der Gase Helium, Argon,
Stickstoff und Sauerstoff umfasst.

4. System zur Durchführung einer Plasmabehandlung von Atemwegsinfektionen
gemäß Anspruch 2,
wobei das Zuführungselement (190) einen Endobronchialschlauch umfasst.

5. System zur Durchführung einer Plasmabehandlung von Atemwegsinfektionen
gemäß Anspruch 2,
wobei das Zuführungselement (190) eine Nasenkanüle oder eine Maske umfasst.

6. System zur Durchführung einer Plasmabehandlung gemäß Anspruch 2,
wobei die Zufuhrgasquelle (150) ein Beatmungsgerät oder eine Vorrichtung zur kontinuierlichen positiven Atemwegsdruckbehandlung umfasst.

7. System zur Durchführung einer Plasmabehandlung von Atemwegsinfektionen gemäß Anspruch 6,
wobei das Zufuhrgas ein Gemisch aus Luft und Sauerstoff umfasst.

8. System zur Durchführung einer Plasmabehandlung von Atemwegsinfektionen gemäß Anspruch 1, wobei der elektrische Energiegenerator (300) so konfiguriert ist, dass er mit einer Frequenz im Bereich von 10 kHz bis 200 kHz und einer Ausgangsspitzen-Spannung im Bereich von 3 kV bis 6 kV arbeitet.

9. System zur Durchführung einer Plasmabehandlung gemäß Anspruch 1, wobei der elektrische Energiegenerator (300) elektrische Energie mit einer Frequenz innerhalb von 25 kHz von 40 kHz, 100 kHz und 200 kHz erzeugt.

10. System zur Durchführung einer Plasmabehandlung von Atemwegsinfektionen gemäß Anspruch 1, wobei der elektrische Energiegenerator (300) einen Hochfrequenz-Elektrochirurgiegenerator und einen Niederfrequenzwandler umfasst.

11. System zur Durchführung einer Plasmabehandlung von Atemwegsinfektionen gemäß Anspruch 1, wobei der elektrische Energiegenerator (300) umfasst:
ein Leistungsmodul (350);
eine CPU (310) zur Steuerung des Leistungsmoduls (350);
einen mit der CPU (310) verbundenen Speicher (311); und
eine mit der CPU (311) verbundene Stromversorgung (302).

12. System zur Durchführung einer Plasmabehandlung von Atemwegsinfektionen gemäß Anspruch 11, wobei der elektrische Energiegenerator (300) ferner umfasst:
ein Touchscreen-Display (420);
eine mit dem Touchscreen-Display verbundene Steuerung; und
eine grafische Benutzeroberfläche, die so konfiguriert ist, dass sie Daten auf dem Touchscreen-Display (420) anzeigt und Eingaben von einem Benutzer über das Touchscreen-Display (420) empfängt.

13. System zur Durchführung einer Plasmabehandlung von Atemwegsinfektionen gemäß Anspruch 11, wobei der elektrische Energiegenerator (300) ferner umfasst:
ein Gasmodul (1000).

14. System zur Durchführung einer Plasmabehandlung von Atemwegsinfektionen gemäß Anspruch 13,
wobei die Quelle für ein Trägergas (110) mit dem Gasmodul (1000) verbunden ist (1000) verbunden ist und das Gasmodul (1000) einen Fluss des Trägergases zu dem Mischer (140) steuert.

15. System zur Durchführung einer Plasmabehandlung von Infektionen der Atemwege gemäß Anspruch 14,
wobei der erste Befeuchter (130) zwischen dem Gasmodul (1000) und dem Mischer (140) angeschlossen ist.

16. System zur Durchführung einer Plasmabehandlung von Atemwegsinfektionen gemäß Anspruch 14,
wobei der erste Befeuchter (130) zwischen dem Gasmodul (1000) und der Trägergasquelle (110) angeschlossen ist.

17. System zur Durchführung einer Plasmabehandlung von Atemwegsinfektionen gemäß Anspruch 1, wobei der erste Befeuchter (130) so konfiguriert ist, dass er ein von der Trägergasquelle (110) strömendes Trägergas auf mindestens 70 % Luftfeuchtigkeit befeuchtet, und der zweite Befeuchter (130) so konfiguriert ist, dass er ein von der Zufuhrgasquelle (150) strömendes Zufuhrgas auf mindestens 50 % Luftfeuchtigkeit befeuchtet.

18. System zur Durchführung einer Plasmabehandlung von Atemwegsinfektionen gemäß Anspruch 1, wobei der erste Befeuchter (130) so konfiguriert ist, dass er ein aus der Trägergasquelle (110) strömendes Trägergas auf 100 % Luftfeuchtigkeit befeuchtet, und der zweite Befeuchter (130) so konfiguriert ist, dass er ein aus der Zufuhrgasquelle (150) strömendes Zufuhrgas auf mindestens 50 % Luftfeuchtigkeit befeuchtet.

19. System zur Durchführung einer Plasmabehandlung von Atemwegsinfektionen gemäß Anspruch 1, das ferner umfasst:
eine Heliumquelle (110), die mit einem Eingang des ersten Befeuchters verbunden ist; und
eine Luftquelle (150), die mit einem Eingang des zweiten Befeuchters verbunden ist.

## Revendications

1. Système pour effectuer un traitement par plasma d'infections respiratoires, comprenant :
un générateur d'énergie électrique (300) configuré pour générer de l'énergie électrique afin de plasmatiser un gaz vecteur en un plasma ; et un mélangeur à décharge à barrière diélectrique (140) comprenant : une chambre intérieure formée d'un diélectrique, ladite chambre intérieure comportant une première entrée (202) configurée pour être reliée de manière fluidique à une source de gaz vecteur humidifié (110), une deuxième entrée (204) configurée pour être reliée à une source de gaz d'alimentation humidifié (150), et une sortie (206) configurée pour être reliée à un élément de distribution (190) ; une électrode active (230) à l'intérieur de ladite chambre intérieure et connectée à une sortie électrique dudit générateur d'énergie électrique ; et une électrode extérieure (220) connectée à une masse ; dans lequel un plasma est généré dans ladite chambre intérieure lorsque de l'énergie électrique est fournie par ledit générateur d'énergie électrique (300) à ladite électrode intérieure (230) tandis que le gaz d'alimentation humidifié et le gaz vecteur humidifié s'écoulent dans ladite chambre intérieure, et comprenant en outre : un premier humidificateur (130) relié de manière fluidique à ladite première entrée (202) de ladite chambre dans ledit ensemble à décharge à barrière diélectrique (140) ; et un deuxième humidificateur (130) relié de manière fluidique à ladite deuxième entrée (204) de ladite chambre dans ledit ensemble à décharge à barrière diélectrique (140).

2. Système pour effectuer un traitement par plasma d'infections respiratoires selon la revendication 1,
comprenant en outre :
une source de gaz vecteur (110) ;
dans lequel le premier humidificateur (130) est relié à ladite source de gaz vecteur (110) ;
une source de gaz d'alimentation (150) ;
dans lequel le deuxième humidificateur (130) est relié à ladite source de gaz d'alimentation (150) ; et
un élément de distribution de fluide (190) relié à une sortie (206) dudit mélangeur (140)
pour distribuer les espèces réactives générées dans ledit mélangeur (140) à un patient.

3. Système pour effectuer un traitement par plasma d'infections respiratoires selon la revendication 2,
dans lequel ledit gaz vecteur comprend au moins l'un parmi l'hélium, l'argon, l' azote et l'oxygène.

4. Système pour effectuer un traitement par plasma d'infections respiratoires selon la revendication 2,
dans lequel ledit élément de distribution (190) comprend un tube endobronchique.

5. Système pour effectuer un traitement par plasma d'infections respiratoires selon la revendication 2,
dans lequel ledit élément de distribution (190) comprend une canule nasale ou un masque.

6. Système pour effectuer un traitement par plasma selon la revendication 2,
dans lequel ladite source de gaz d'alimentation (150) comprend un ventilateur ou un dispositif à pression positive continue.

7. Système pour effectuer un traitement par plasma d'infections respiratoires selon la revendication 6,
dans lequel ledit gaz d'alimentation comprend un mélange d'air et d'oxygène.

8. Système pour effectuer un traitement par plasma d'infections respiratoires selon la revendication 1, dans lequel ledit générateur d'énergie électrique (300) est configuré pour fonctionner à une fréquence comprise entre 10 kHz et 200 kHz et avec une tension de crête de sortie comprise entre 3 kV et 6 kV.

9. Système pour effectuer un traitement par plasma selon la revendication 1, dans lequel ledit générateur d'énergie électrique (300) génère une énergie électrique ayant une fréquence comprise dans une plage de 25 kHz autour de l'une des fréquences 40 kHz, 100 kHz et 200 kHz.

10. Système pour effectuer un traitement par plasma d'infections respiratoires selon la revendication 1, dans lequel ledit générateur d'énergie électrique (300) comprend un générateur électrochirurgical à haute fréquence et un convertisseur à basse fréquence.

11. Système pour effectuer un traitement par plasma d'infections respiratoires selon la revendication 1, dans lequel ledit générateur d'énergie électrique (300) comprend :
un module d'alimentation (350) ;
une unité centrale (310) pour commander ledit module d'alimentation (350) ;
une mémoire (311) connectée à ladite unité centrale (310) ; et
une alimentation électrique (302) connectée à ladite unité centrale (311).

12. Système pour effectuer un traitement par plasma d'infections respiratoires selon la revendication 11, dans lequel ledit générateur d'énergie électrique (300) comprend en outre :
un écran tactile (420) ;
un contrôleur connecté audit écran tactile ; et
une interface utilisateur graphique configurée pour afficher des données sur ledit écran tactile (420) et recevoir des entrées d'un utilisateur via ledit écran tactile (420).

13. Système pour effectuer un traitement par plasma d'infections respiratoires selon la revendication 11, dans lequel ledit générateur d'énergie électrique (300) comprend en outre :
un module de gaz (1000).

14. Système pour effectuer un traitement par plasma d'infections respiratoires selon la revendication 13,
dans lequel ladite source de gaz vecteur (110) est connectée audit module de gaz (1000) module de gaz (1000) et ledit module de gaz (1000) contrôle un flux dudit gaz vecteur vers ledit mélangeur (140).

15. Système pour effectuer un traitement par plasma d'infections respiratoires selon la revendication 14,
dans lequel ledit premier humidificateur (130) est connecté entre ledit module de gaz (1000) et ledit mélangeur (140).

16. Système pour effectuer un traitement par plasma d'infections respiratoires selon la revendication 14,
dans lequel ledit premier humidificateur (130) est connecté entre ledit module de gaz (1000) et ladite source de gaz vecteur (110).

17. Système pour effectuer un traitement par plasma d'infections respiratoires selon la revendication 1, dans lequel ledit premier humidificateur (130) est configuré pour humidifier un gaz vecteur s'écoulant de ladite source de gaz vecteur (110) à au moins 70 % d'humidité et ledit deuxième humidificateur (130) est configuré pour humidifier un gaz d'alimentation s'écoulant de ladite source de gaz d'alimentation (150) à au moins 50 % d'humidité.

18. Système pour effectuer un traitement par plasma d'infections respiratoires selon la revendication 1, dans lequel ledit premier humidificateur (130) est configuré pour humidifier un gaz vecteur s'écoulant depuis ladite source de gaz vecteur (110) à 100 % d'humidité et ledit deuxième humidificateur (130) est configuré pour humidifier un gaz d'alimentation s'écoulant depuis ladite source de gaz d'alimentation (150) à au moins 50 % d'humidité.

19. Système pour effectuer un traitement par plasma d'infections respiratoires selon la revendication 1, comprenant en outre :
une source d'hélium (110) connectée à une entrée dudit premier humidificateur ; et
une source d'air (150) connectée à une entrée dudit deuxième humidificateur.
